# EUROPÄISCHE PATENTSCHRIFT

(11) **EP 3 011 996 B1**
(45) Veröffentlichungstag und Bekanntmachung des Hinweises auf die Patenterteilung: **26.08.2020**
(21) Anmeldenummer: 15002843.9
(22) Anmeldetag: 05.10.2015
(51) Int. Cl.: A61N 2/06, A61N 2/00

(54) **MAGNETVORRICHTUNG**
MAGNETIC DEVICE
DISPOSITIF MAGNETIQUE

(30) Priorität: 24.10.2014 EP 14003626
(43) Veröffentlichungstag der Anmeldung: 27.04.2016
(73) Patentinhaber: Grander GmbH, 6373 Jochberg (AT)
(72) Erfinder: Grander, Heribert, 6370 Kitzbühel (AT); Grander, Johann, 6373 Jochberg (AT)
(74) Vertreter: Richter, Thomas Kurt Reinhold

(56) Entgegenhaltungen:
- WO-A1-82/03177
- US-A1- 2009 082 690
- US-A1- 2014 194 668

## Beschreibung

Die Erfindung betrifft eine Magnetvorrichtung gemäß den angefügten Ansprüchen 1-23.

Die US 2009/0082690 A1 offenbart mehrere, zum Teil sehr unterschiedlich aufgebaute Magnetvorrichtungen, mit deren Hilfe ein psychischer Zustand eines Menschen positiv beeinflußt werden kann. Beispielsweise finden die in der US 2009/0082690 A1 offenbarten Magnetvorrichtungen Anwendung bei der Behandlung von Depression, Eßstörungen, Schlafstörungen, Tinnitus, der Alzheimer-Krankheit und anderen Erkrankungen. Bei einigen der in der US 2009/0082690 A1 offenbarten Magnetvorrichtungen sind mehrere zylindrische Magneten vorgesehen, von denen jeder einzelne aus zwei länglichen halbzylinderförmigen Teilschalen besteht, wobei die eine der beiden Teilschalen den Nordpol und die andere der beiden Teilschalen den Südpol des jeweiligen Magneten bildet. Diese eben genannten Magneten sind zueinander parallel orientiert angeordnet, und jeder einzelne dieser Magneten weist jeweils ein Loch auf, das sich mittig entlang seiner Längsachse durch den jeweiligen Magneten hindurch erstreckt. Dieses Loch dient zur Aufnahme eines Antriebsschaftes oder eines Zentrierstiftes. Der Antriebsschaft bzw. der Zentrierstift erstreckt sich beidseitig über die Stirnwände des jeweiligen Magneten hinaus und ist an seinen Enden jeweils so gelagert, daß der jeweilige Magnet keinerlei Wandberührung hat und mittels eines Antriebsmechanismus, welcher unter anderem den Antriebsschaft bzw. den Zentrierstift umfaßt, um seine Längsachse herum gedreht werden kann.

Eine ähnliche gattungsgemäße Magnetvorrichtung ist in der WO 82/03177 A1 offenbart. Derartige gattungsgemäße Magnetvorrichtungen mit allerdings jeweils genau drei stabförmigen Permanentmagneten sind aus dem Stand der Technik unter der Handelsbezeichnung SANOMAG bekannt. Ihr weltweiter Vertrieb erfolgt bereits seit 1980, wobei die im Stand der Technik unter der Handelsbezeichnung SANOMAG vertriebenen Magnetvorrichtungen außer den genannten stabförmigen Permanentmagneten zusätzlich stets auch mindestens ein Stahlseil und in den allermeisten Fällen zwei Stahlseile aufwiesen, wie weiter unten noch genauer beschrieben wird. Laut vielfachen Anwenderberichten werden diese gattungsgemäßen Magnetvorrichtungen samt den im Stand der Technik zusätzlich stets vorhandenen Stahlseilen erfolgreich für Therapiezwecke eingesetzt, und zwar zur Behandlung von Gelenkschmerzen, Kopfschmerzen, Migräne, Rheumaschmerzen und Schlafstörungen. Außerdem gibt es Anwenderberichte darüber, daß derartige Magnetvorrichtungen ganz allgemein das Wohnklima verbessern. Vereinzelt wurde sogar über den Einsatz derartiger gattungsgemäßer Magnetvorrichtungen samt dem bzw. den im Stand der Technik zusätzlich stets vorhandenen Stahlseil(en) zur Unterstützung der Trocknung von Mauerwerk berichtet.

Die theoretische Deutung der genauen Wirkungsweise dieser aus dem Stand der Technik bekannten und seit Jahrzehnten erfolgreich im praktischen therapeutischen Einsatz angewendeten und vielfach erprobten gattungsgemäßen Magnetvorrichtungen befindet sich noch im Stadium der Erforschung und ist Gegenstand wissenschaftlicher Untersuchungen und Diskussionen. Als gesichert gilt dabei jedenfalls, daß der menschliche Körper neben seiner biologischchemischen Wesenheit auch ein nichtstatisches, mithin also zeitlich und räumlich veränderliches, elektromagnetisches System ist, so daß ohne Zweifel eine gewisse Wechselwirkung zwischen dem menschlichen Körper und dem Magnetfeld der Magnetvorrichtung stattfindet. Der menschliche Körper kann zu diesem Zweck bei den aus dem Stand der Technik unter der Handelsbezeichnung SANOMAG bekannten Magnetvorrichtungen entweder in unmittelbaren Kontakt mit etwaig an den Enden der beiden Stahlseile befestigten Griffelementen gebracht und auf diese Weise direkt in den magnetischen Kreis der Magnetvorrichtung eingeschlossen werden oder sich einfach in das magnetische Streufeld der Magnetvorrichtung begeben.

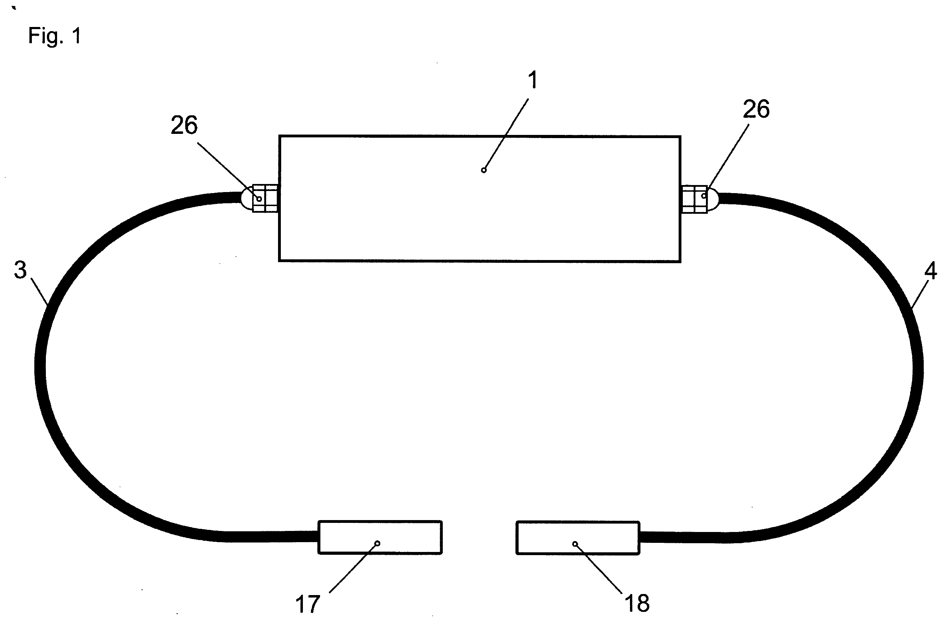

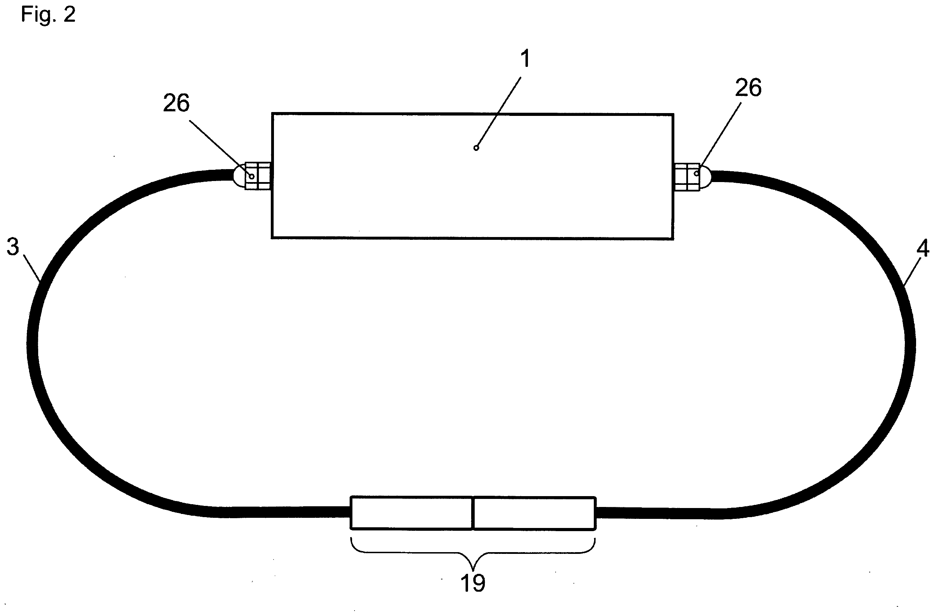

Schematische Gesamt-Außenansichten von zwei aus dem Stand der Technik bekannten Beispielen gattungsgemäßer Magnetvorrichtungen sind schematisch in Fig. 1 bzw. Fig. 2 dargestellt. Beide Magnetvorrichtungen aus dem Stand der Technik weisen ein Gehäuse 1 auf. Mit dem Gehäuse 1 mechanisch gekoppelt sind ein Ende eines ersten Stahlseils 3 und ein Ende eines zweiten Stahlseils 4. Jedes der beiden Stahlseile 3, 4 besteht aus mehreren verdrillten Stahllitzen.

Bei dem Beispiel von Fig. 1 endet jedes der beiden Stahlseile 3, 4 an seinem jeweils anderen Ende in jeweils einem der bereits oben kurz erwähnten Griffelemente 17, 18. In jedem dieser beiden Griffelemente 17, 18 ist jeweils ein Permanentmagnet angeordnet. Zwecks therapeutischer Anwendung der Magnetvorrichtung werden die beiden Griffelemente 17, 18 in Kontakt mit einem zu behandelnden Lebewesen gebracht. Dies kann beispielsweise einfach derart ausgeführt werden, daß ein zu behandelnder Mensch mit jeder Hand jeweils eines der beiden Griffelemente 17, 18 ergreift und sich auf diese Weise unmittelbar in den magnetischen Kreis der Magnetvorrichtung einschaltet.

Bei dem Beispiel von Fig. 2 sind die beiden Stahlseile 3, 4 mit ihren jeweiligen Griffelementen aneinandergefügt. Normalerweise genügt hierfür schon allein die Magnetkraft der in den beiden Griffelementen befindlichen und entsprechend gepolten Permanentmagnete, um die jeweiligen Stirnseiten der beiden Griffelemente aneinander zu fixieren. Eine solche Ausführung der gattungsgemäßen Magnetvorrichtung wird beispielsweise verwendet, um auf einen Wohnraum einzuwirken oder - bei entsprechender Verlängerung der Stahlseile 3, 4 - ein ganzes Haus zu umfassen. Sofern die Gefahr besteht, daß dabei Zug auf die Stahlseile 3, 4 ausgeübt wird, etwa durch spielende Kinder, werden die beiden aneinandergefügten Griffelemente zusätzlich noch mittels einer Sicherungseinrichtung 19 aneinander fixiert, so wie es in Fig. 2 dargestellt ist. Als Sicherungseinrichtung 19 kann z. B. ein Klebeband verwendet werden.

Bei einer besonderen Modifikation des in Fig. 2 gezeigten Beispiels einer Magnetvorrichtung aus dem Stand der Technik sind gar keine Griffelemente vorhanden, und es gibt auch nur ein durchgehendes Stahlseil, welches man rein formal als aus zwei Abschnitten bestehend betrachten kann, wobei der eine Stahlseilabschnitt dem ersten Stahlseil 3 und der zweite Stahlseilabschnitt dem zweiten Stahlseil 4 entspricht. Aufgrund des durchgehenden Stahlseils entfällt bei dieser besonderen Modifikation die Sicherungseinrichtung 19. Auch eine solche Ausführung der gattungsgemäßen Magnetvorrichtung wird beispielsweise dazu verwendet, mit dem Stahlseil ein ganzes Haus zu umfassen.

Ferner ist es möglich, zwei, drei oder noch mehr dieser Magnetvorrichtungen in Reihe zusammenzuschalten. Zwischen den jeweiligen Gehäusen erstreckt sich dann jeweils ein Stahlseil. Auch eine solche Ausführung der gattungsgemäßen Magnetvorrichtung wird beispielsweise dazu verwendet, ein ganzes Haus zu umfassen.

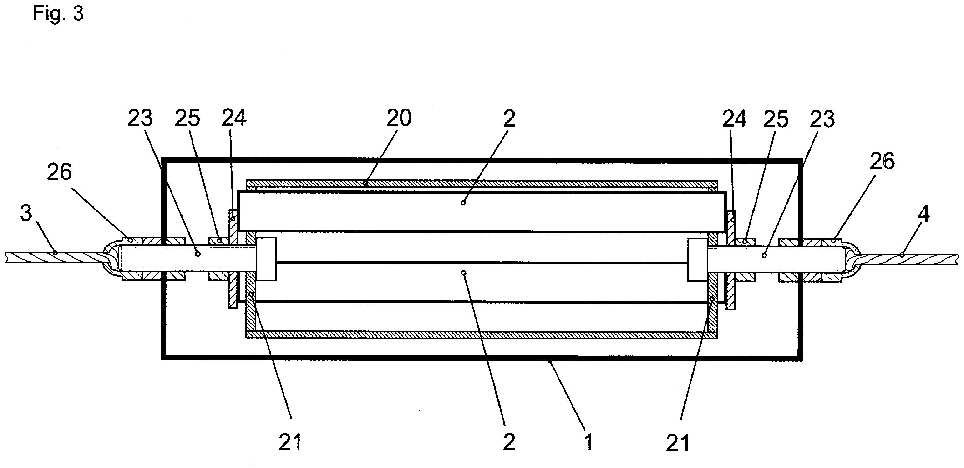

Eine schematische und unvollständige Schnittdarstellung des Gehäuses 1 der aus dem Stand der Technik bekannten Magnetvorrichtungen von Fig. 1 bzw. von Fig. 2 ist in Fig. 3 gezeigt. In dem von dem Gehäuse 1 umschlossenen Raum befindet sich eine Stahlröhre 20, in welcher jeweils parallel zueinander drei um jeweils 120° radial versetzt angeordnete stabförmige Permanentmagnete 2 angeordnet sind. Der Übersichtlichkeit halber wurde in der rein schematischen Darstellung von Fig. 3 einer der drei stabförmigen Permanentmagnete weggelassen, so daß dort nur zwei stabförmige Permanentmagnete 2 zu sehen sind. Alle drei stabförmigen Permanentmagnete 2 sind magnetisch gleichsinnig orientiert angeordnet, d. h. die Nordpole aller drei stabförmigen Permanentmagnete 2 weisen jeweils in ein und dieselbe Richtung, und die Südpole aller drei stabförmigen Permanentmagnete 2 weisen jeweils gemeinsam in die entgegengesetzte Richtung.

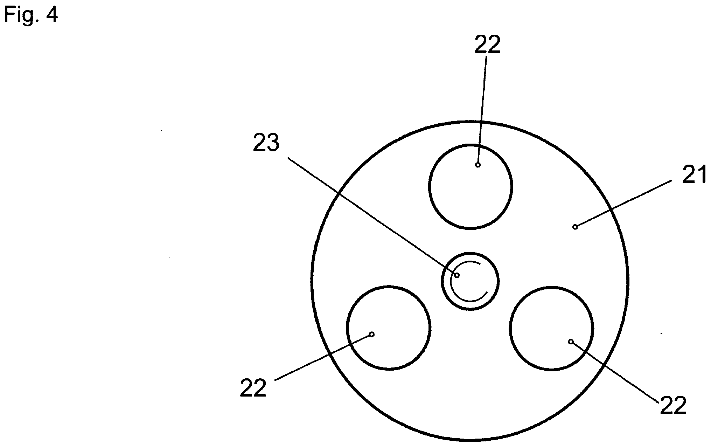

Auf jede der beiden Stirnseiten der Stahlröhre 20 ist jeweils eine ungefähr 3 mm dicke erste Stahlscheibe 21 geschweißt. Jede der beiden ersten Stahlscheiben 21 (siehe Fig. 4) weist drei Löcher 22 zum Hindurchstecken der stabförmigen Permanentmagnete 2 auf. Außerdem befindet sich jeweils im Zentrum jeder der beiden ersten Stahlscheiben 21 ein weiteres Loch, durch das eine Schraube 23 gesteckt ist. Jede der beiden Schrauben 23 ist mit der ihr jeweils zugehörigen ersten Stahlscheibe 21 verschweißt.

Alle drei stabförmigen Permanentmagnete 2 sind jeweils gleich lang und dabei etwas länger als die Stahlröhre 20. Zur Montage werden die stabförmigen Permanentmagnete 2 jeweils in gleicher magnetischer Orientierung durch die Löcher 22 in die Stahlröhre 20 hineingeschoben. Anschließend wird auf jeder der beiden Stirnseiten der aus der Stahlröhre 20 beidseitig jeweils ungefähr 1 mm bis 2 mm herausragenden stabförmigen Permanentmagnete 2 jeweils eine ungefähr 2 mm dicke zweite Stahlscheibe 24 aufgesetzt. Jede dieser beiden zweiten Stahlscheiben 24 hat genau ein Loch, welches sich jeweils in der Mitte der zweiten Stahlscheibe 24 befindet. Über dieses Loch wird die jeweilige zweite Stahlscheibe 24 auf die jeweils zugehörige Schraube 23 aufgesteckt. Jede der beiden zweiten Stahlscheiben 24 hat einen kleineren Durchmesser als jede der beiden ersten Stahlscheiben 21 und deckt eine jeweilige Stirnseite jedes der stabförmigen Permanentmagnete 2 nur zum Teil ab. Jede der beiden zweiten Stahlscheiben 24 ist jedoch groß genug, um alle vorhandenen stabförmigen Permanentmagnete 2 sicher fixieren zu können. Die Befestigung der zweiten Stahlscheibe 24 erfolgt jeweils mittels zweier Kontermuttern 25, die jeweils auf der von den stabförmigen Permanentmagneten 2 abgewandten Seite der zweiten Stahlscheibe 24 auf die jeweilige Schraube 23 geschraubt werden.

Jede der beiden Schrauben 23 lagert an ihrem von den stabförmigen Permanentmagneten 2 abgewandten Ende in dem die Stahlröhre 20 umschließenden Gehäuse 1. Eine der beiden Schrauben 23 ist mit einem Ende des ersten Stahlseils 3 verlötet oder verpreßt oder mittels Spannkeils verbunden, und die andere der beiden Schrauben 23 ist mit einem Ende des zweiten Stahlseils 4 verlötet oder verpreßt oder mittels Spannkeils verbunden. Wichtig ist eine schlüssige, dem Magnetfluß entsprechende Verbindung. Die jeweilige Lötstelle bzw. Preßstelle ist jeweils durch eine Überwurfmutter 26 gesichert.

Der Erfindung liegt die Aufgabe zugrunde, eine neue Variante einer Magnetvorrichtung bereitzustellen, bei der gegenüber den aus dem Stand der Technik unter der Handelsbezeichnung SANOMAG bekannten Magnetvorrichtungen das Zusammenbauen der Magnetvorrichtung vereinfacht wird.

Die Aufgabe der Erfindung wird durch eine Vorrichtung mit den Merkmalen eines der Patentansprüche 1, 2, 5 oder 6 gelöst. Vorteilhafte Ausführungsformen dieser Vorrichtung sind in den Unteransprüchen 3, 4 und 7 bis 23 beschrieben. Durch das Vorsehen der beiden jeweils einteilig ausgebildeten, stählernen Sockelelemente der erfindungsgemäßen Magnetvorrichtung mit den in jenen einteilig ausgebildeten, stählernen Sockelelementen vorgesehenen Vertiefungen zur Aufnahme von Stirnseiten der stabförmigen Permanentmagnete wird gegenüber den aus dem Stand der Technik unter der Handelsbezeichnung SANOMAG bekannten Magnetvorrichtungen die Anzahl der Einzelbauteile verringert, was den vorteilhaften Effekt bewirkt, daß das Zusammenbauen der erfindungsgemäßen Magnetvorrichtung im Vergleich zu der aus dem Stand der Technik unter der Handelsbezeichnung SANOMAG bekannten Magnetvorrichtung erheblich vereinfacht wird. Noch deutlicher wird dieser besonders positive Effekt des vereinfachten Zusammenbauens bei solchen speziellen Ausführungsformen erfindungsgemäßer Magnetvorrichtungen, die, anders als die aus dem Stand der Technik bekannten Magnetvorrichtungen, gar keine Stahlseile oder ein oder zwei einfach lösbar befestigbare Stahlseile aufweisen. Wie sich in Experimenten gezeigt hat, gewährleisten die beiden einteilig ausgebildeten, stählernen Sockelelemente mit den in jenen einteilig ausgebildeten, stählernen Sockelelementen vorgesehenen Vertiefungen zur Aufnahme von Stirnseiten der stabförmigen Permanentmagnete einen gegenüber der aus dem Stand der Technik bekannten Magnetvorrichtung verbesserten weil ganz besonders gut konzentrierten magnetischen Fluß. Die beiden einteilig ausgebildeten, stählernen Sockelelemente in der erfindungsgemäßen Magnetvorrichtung werden daher auch gelegentlich als "Konzentratorplatten" bezeichnet. Somit ist es bei der erfindungsgemäßen Magnetvorrichtung nicht einmal unbedingt notwendig, überhaupt Stahlseile vorzusehen, obwohl es auch Ausführungsformen der erfindungsgemäßen Magnetvorrichtung mit Stahlseilen gibt. Bei Ausführungsformen der erfindungsgemäßen Magnetvorrichtung ohne Stahlseile ist der Magnetfluß dementsprechend nicht mehr an die Stahlseile gebunden und kann sich frei im Raum entfalten, was besser für stationäre Anwendungen ist. Eine erfindungsgemäße Magnetvorrichtung in ihrer allereinfachsten Ausführungsform ganz ohne Stahlseile kann einfach in einem Raum, beispielsweise in einem Wohnraum oder in einer Stallung, aufgestellt werden und dient dann dort zur Raumharmonisierung, was ein verbessertes Wohlbefinden der diesen Raum bewohnenden bzw. in diesem Raum untergebrachten Menschen bzw. Tiere zur Folge hat. Vorteilhafte und bevorzugte Ausführungsformen der erfindungsgemäßen Magnetvorrichtung sind Gegenstand der Ansprüche 3, 4, 7 bis 23.

Die in den beiden jeweils einteilig ausgebildeten, stählernen Sockelelementen der erfindungsgemäßen Magnetvorrichtung stets vorgesehenen Vertiefungen zur Aufnahme von Stirnseiten der stabförmigen Permanentmagnete sowie das gemäß verschiedener spezieller Ausführungsformen der erfindungsgemäßen Magnetvorrichtung vorgesehene unmittelbare Hineinstecken von Endabschnitten eines Stahlseils oder von jeweils einem Endabschnitt von jeweils zwei Stahlseilen in die genannten einteiligen Sockelelemente gewährleisten einen gegenüber der aus dem Stand der Technik bekannten Magnetvorrichtung verbesserten magnetischen Fluß in die Stahlseile hinein, weil durch den innigeren Kontakt sowie durch die Verringerung der Anzahl der Einzelbauteile und damit der Anzahl der Bauteilübergänge im Bereich zwischen den stabförmigen Permanentmagneten einerseits und dem jeweiligen Stahlseil andererseits die in diesem Bereich unerwünschte magnetische Streuung verringert wird. Ganz entsprechendes gilt für diejenigen speziellen Ausführungsformen der erfindungsgemäßen Magnetvorrichtung, bei denen zwar ein Stahlseil oder zwei Stahlseile vorgesehen ist bzw. sind, dieses bzw. diese jedoch nicht dauerhaft fest mit den Sockelelementen verbunden ist bzw. sind. Bei derartigen speziellen Ausführungsformen der erfindungsgemäßen Magnetvorrichtung kann zum Beispiel vorgesehen sein, mindestens ein Ende eines Stahlseils in einem magnetisch leitenden Zylinder zu befestigen, welcher so ausgebildet ist, daß er an einem der Sockelelemente lösbar befestigbar ist, indem er durch die magnetische Kraft der stabförmigen Permanentmagnete auf das Sockelelement gezogen wird. So können das Stahlseil oder die Stahlseile leicht an die Sockelelemente angeschlossen, aber auch wieder rasch und ohne großen Aufwand von den Sockelelementen abgenommen werden, und die so gestalteten speziellen Ausführungsformen der erfindungsgemäßen Magnetvorrichtung können je nach wechselndem Einsatzwunsch unkompliziert an sich ändernde Einsatzbedingungen angepaßt und entweder mit aufgesetztem Stahlseil bzw. aufgesetzten Stahlseilen oder gänzlich ohne Stahlseil verwendet werden. Auch lassen sich derartige spezielle Ausführungsformen der erfindungsgemäßen Magnetvorrichtung besonders einfach zum Transport verpacken oder tragen, wenn das Stahlseil oder die Stahlseile abgezogen sind und folglich separat verpackt oder getragen werden können. Derartige besonders flexible Ausführungsformen der erfindungsgemäßen Magnetvorrichtung sind ganz besonders bevorzugt, und sie sind beispielsweise Gegenstand der Ansprüche 14 und 15. Allerdings muß erwähnt werden, daß das Stahlseil oder die Stahlseile auch auf andere Weise als mit Hilfe von magnetisch leitenden Zylindern an dem Sockelelement bzw. an den Sockelelementen lösbar befestigbar gestaltet werden können. Sowohl Anspruch 1 als auch Anspruch 13 bieten insofern einen sehr breiten Gestaltungsspielraum für diverse weitere Ausführungsformen erfindungsgemäßer Magnetvorrichtungen, was der Fachmann ohne weiteres anerkennen wird.

Besonders bevorzugt ist in jedem Falle, und zwar unabhängig davon, ob Stahlseile vorgesehen sind oder nicht, dabei die Ausführungsform gemäß Anspruch 3. Gestalt und Größe der Vertiefungen in den Sockelelementen sind bei dieser Ausführungsform eng auf die Gestalt und Größe der stabförmigen Permanentmagnete abgestimmt. Bei dieser Ausführungsform gibt es ein nur noch sehr geringes Spiel zwischen dem stabförmigen Permanentmagneten und der ihn jeweils nach Art eines Bechers aufnehmenden Vertiefung. So wird ein besonders guter magnetischer Fluß zwischen den stabförmigen Permanentmagneten und den etwaig vorhandenen Stahlseilen bzw., sofern kein Stahlseil vorgesehen ist, ein besonders konzentrierter magnetischer Fluß in den Außenraum hinein erzielt.

Besonders bevorzugt ist ferner die Ausführungsform gemäß Anspruch 10. Zwar wird bei dieser Ausführungsform mit der Röhre ein zusätzliches Bauteil eingeführt, jedoch ist dieses Bauteil einfach zu montieren. Die Röhre wird nämlich beim Montagevorgang lediglich über die vorher schon in eines der beiden Sockelelemente eingesteckten stabförmigen Permanentmagnete gestülpt und bildet dann ihrerseits eine Montagehilfe für das Einstecken der stabförmigen Permanentmagnete in das andere Sockelelement.

Die vorstehend genannte Röhre wird bevorzugt aus nichtmagnetischem Material oder aus nur gering magnetischem Material wie beispielsweise Kunststoff, Aluminium oder Messing gefertigt. Eine solche erfindungsgemäße Materialauswahl wirkt sich positiv auf die Konzentration des magnetischen Flusses in Richtung der etwaig vorhandenen Stahlseile bzw., sofern kein Stahlseil vorhanden ist, in Richtung des Außenraumes aus, weil derartiges Material im Gegensatz zu der in den Magnetvorrichtungen aus dem Stand der Technik verwendeten Stahlröhre den magnetischen Fluß nicht von den stählernen Sockelelementen abzieht.

Besonders bevorzugt sind ferner solche Ausführungsformen der erfindungsgemäßen Magnetvorrichtung, die, wie es beispielsweise bei den Ausführungsformen gemäß Anspruch 4 oder gemäß Anspruch 13 oder gemäß Anspruch 20 vorgesehen ist, ein Gehäuse aufweisen. Allerdings sei an dieser Stelle ausdrücklich betont, daß, obwohl Ausführungsformen der erfindungsgemäßen Magnetvorrichtung mit Gehäuse besonders bevorzugt werden, durchaus auch solche Ausführungsformen der erfindungsgemäßen Magnetvorrichtung existieren, bei denen kein Gehäuse vorgesehen ist.

Ausführungsbeispiele der erfindungsgemäßen Magnetvorrichtung werden nachfolgend anhand von Figuren erläutert. Es zeigt:
- Fig. 1: schematisch eine Gesamt-Außenansicht eines ersten Beispiels einer Magnetvorrichtung aus dem Stand der Technik,
- Fig. 2: schematisch eine Gesamt-Außenansicht eines zweiten Beispiels einer Magnetvorrichtung aus dem Stand der Technik,
- Fig. 3: schematisch einen Längsschnitt durch ein Gehäuse einer Magnetvorrichtung aus dem Stand der Technik,
- Fig. 4: schematisch eine Draufsicht auf eine erste Stahlscheibe an einer Stirnseite einer Stahlröhre in der Magnetvorrichtung von Fig. 3 aus dem Stand der Technik,
- Fig. 5: schematisch eine Gesamt-Außenansicht eines ersten Ausführungsbeispiels einer erfindungsgemäßen Magnetvorrichtung,
- Fig. 6: schematisch eine Gesamt-Außenansicht eines zweiten Ausführungsbeispiels einer erfindungsgemäßen Magnetvorrichtung,
- Fig. 7: schematisch einen Längsschnitt durch ein Gehäuse eines Ausführungsbeispiels einer erfindungsgemäßen Magnetvorrichtung,
- Fig. 8: schematisch eine Seitenansicht des Ausführungsbeispiels der erfindungsgemäßen Magnetvorrichtung von Fig. 7 ohne Röhre, Gehäusedeckel und Stahlseile,
- Fig. 9: einen Querschnitt längs der Schnittlinie B - B aus Fig. 8 mit hinzugefügter Röhre,
- Fig. 10: schematisch eine perspektivische Darstellung des Ausführungsbeispiels von Fig. 7 bzw. Fig. 8 ohne Röhre und Gehäusedeckel,
- Fig. 11: schematisch einen Teil der perspektivischen Darstellung von Fig. 10 mit hinzugefügter Röhre im Querschnitt,
- Fig. 12: schematisch eine Draufsicht auf eine Stirnseite des Gehäuses des Ausführungsbeispiels einer erfindungsgemäßen Magnetvorrichtung von Fig. 7 bzw. Fig. 8 und
- Fig. 13: schematisch einen Längsschnitt durch das Gehäuse eines weiteren Ausführungsbeispiels einer erfindungsgemäßen Magnetvorrichtung.

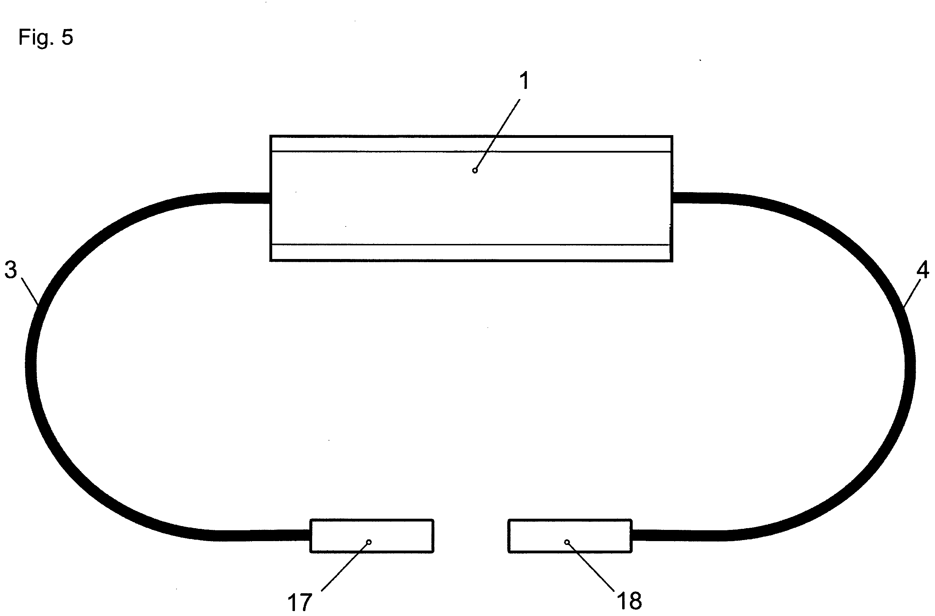

Im folgenden werden unter Bezugnahme auf die Fig. 5 bis 13 einige besonders bevorzugte Ausführungsbeispiele zweier verschiedener Beispielvarianten der erfindungsgemäßen Magnetvorrichtung näher erläutert.

Bei der unter Bezugnahme auf die Fig. 5 bis 12 erläuterten ersten Beispielvariante mit ihren verschiedenen Ausführungsbeispielen handelt sich um eine Beispielvariante der erfindungsgemäßen Magnetvorrichtung, bei der stets mindestens ein Stahlseil vorhanden ist, welches mit einem Ende in einem Sockelelement steckt und dort fixiert ist.

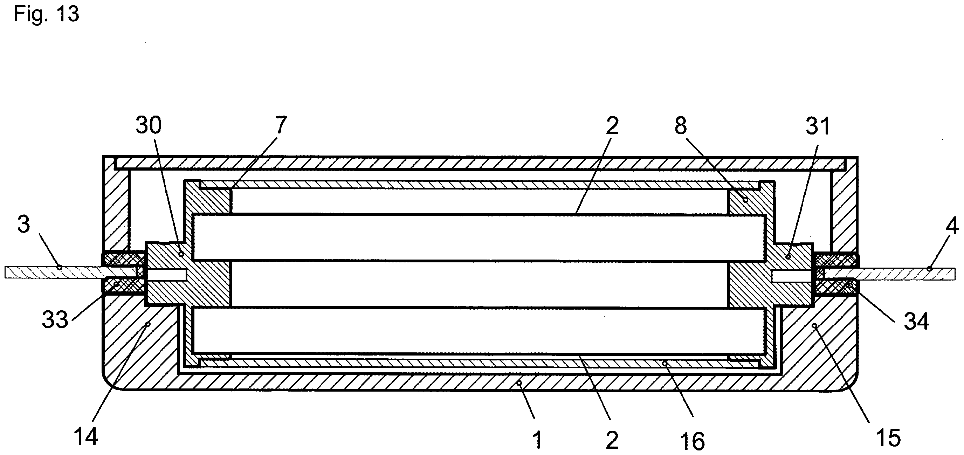

Bei der unter Bezugnahme auf die Fig. 13 erläuterten zweiten Beispielvariante mit ihren verschiedenen Ausführungsbeispielen handelt es sich um eine Beispielvariante der erfindungsgemäßen Magnetvorrichtung, bei der zwar stets auch mindestens ein Stahlseil vorhanden ist, welches jedoch an dem Sockelelement lösbar befestigbar ist, indem es durch die magnetische Kraft stabförmiger Permanentmagnete auf das Sockelelement gezogen wird.

Bereits an dieser Stelle sei darauf hingewiesen, daß bei dem Ausführungsbeispiel gemäß Fig. 13 das Stahlseil bzw. die Stahlseile auch entfernt werden kann bzw. können und die Magnetvorrichtung dann völlig ohne Stahlseil aufgestellt werden kann.

Überhaupt gibt es auch weitere, in den Figuren nicht gesondert dargestellte Ausführungsbeispiele einer dritten Beispielvariante der erfindungsgemäßen Magnetvorrichtung mit allen in Anspruch 1 aufgeführten Merkmalen, wobei jedoch bei den Ausführungsbeispielen dieser dritten Beispielvariante der erfindungsgemäßen Magnetvorrichtung Stahlseile gänzlich fehlen. Derartige Ausführungsbeispiele der erfindungsgemäßen Magnetvorrichtung werden dann bevorzugt rein stationär zur Raumharmonisierung eingesetzt.

Die größte Einsatz- und Anwendungsflexibilität bieten allerdings natürlich die Ausführungsbeispiele gemäß der oben erwähnten zweiten Beispielvariante der erfindungsgemäßen Magnetvorrichtung, bei denen das Stahlseil oder die Stahlseile je nach gewünschtem Einsatzzweck einfach aufgesteckt und auch einfach wieder abgezogen werden kann bzw. können, so daß eine Anpassung der Magnetvorrichtung an wechselnde Einsatzbedingungen schnell und problemlos erfolgen kann. Darüber hinaus läßt sich die Magnetvorrichtung bequemer von einem Einsatzort zu einem anderen Einsatzort transportieren, wenn das Stahlseil oder die Stahlseile abgezogen wurden. Einige Ausführungsbeispiele dieser zweiten Beispielvariante der erfindungsgemäßen Magnetvorrichtung werden weiter unten mit Bezug auf die Fig. 13 näher erläutert.

Vorher wenden wir uns jedoch den Fig. 5 bis 12 und damit Ausführungsbeispielen der ersten Beispielvariante der erfindungsgemäßen Magnetvorrichtung zu.

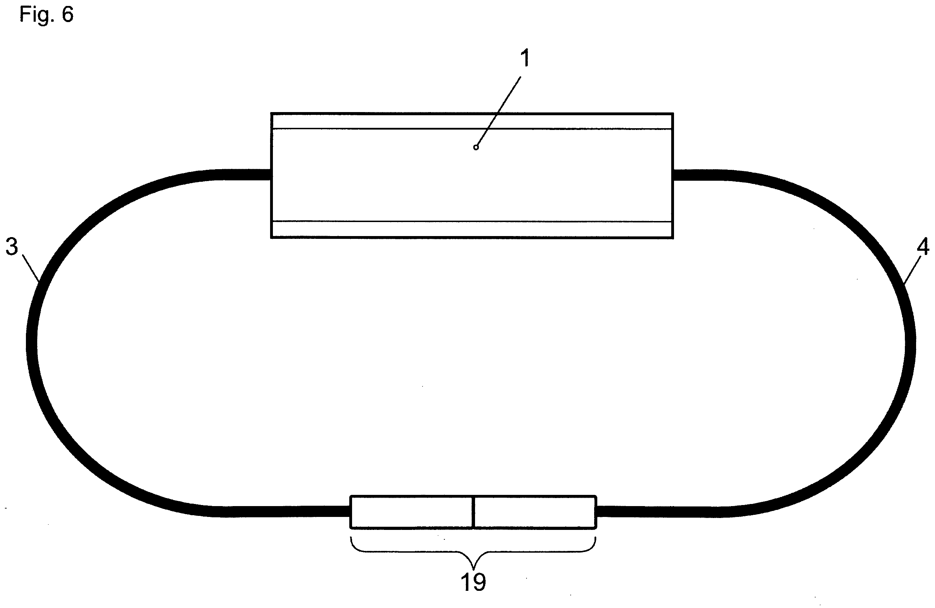

Die im folgenden nun zunächst zu beschreibenden und in den Fig. 5 bis 12 dargestellten Ausführungsbeispiele der ersten Beispielvariante erfindungsgemäßer Magnetvorrichtungen unterscheiden sich schon in ihren in den Fig. 5 bzw. 6 gezeigten schematischen Gesamt-Außenansichten von den bereits oben mit Bezug auf die Fig. 1 bzw. 2 beschriebenen Magnetvorrichtungsbeispielen aus dem Stand der Technik.

Zwar weisen auch die vorliegend zu beschreibenden Ausführungsbeispiele der erfindungsgemäßen Magnetvorrichtung ein Gehäuse 1 auf. Von dem genannten Gehäuse 1 aus erstrecken sich auch bei den Ausführungsbeispielen der erfindungsgemäßen Magnetvorrichtungen gemäß den Fig. 5 bis 12 ein erstes Stahlseil 3 und ein zweites Stahlseil 4.

Bei einem ersten, in Fig. 5 dargestellten Ausführungsbeispiel der erfindungsgemäßen Magnetvorrichtung endet jedes der beiden Stahlseile 3, 4 außerhalb des Gehäuses 1 in jeweils einem Griffelement 17, 18. In jedem dieser beiden Griffelemente 17, 18 ist jeweils ein Permanentmagnet angeordnet. Das entspricht insoweit dem in Fig. 1 gezeigten ersten Beispiel einer Magnetvorrichtung aus dem Stand der Technik.

Bei einem zweiten, in Fig. 6 dargestellten Ausführungsbeispiel der erfindungsgemäßen Magnetvorrichtung sind die mit den Griffelementen versehenen Enden der beiden Stahlseile 3, 4 aneinandergefügt und mittels einer Sicherungseinrichtung 19 aneinander fixiert. Als Sicherungseinrichtung 19 kann beispielsweise ein Klebeband verwendet werden. Das entspricht insoweit dem in Fig. 2 gezeigten zweiten Beispiel einer Magnetvorrichtung aus dem Stand der Technik. Wird zumindest eines der beiden Stahlseile 3, 4 genügend lang gewählt, so kann dieses Ausführungsbeispiel der erfindungsgemäßen Magnetvorrichtung derart eingesetzt werden, daß die Magnetvorrichtung ein ganzes Haus umschließt.

Im Gegensatz zum Stand der Technik beginnen die beiden Stahlseile 3, 4 bei den in den Fig. 5 bis 12 dargestellten Ausführungsbeispielen erfindungsgemäßer Magnetvorrichtungen jedoch nicht erst außerhalb des Gehäuses 1, sondern sie reichen beide bis in das Innere des Gehäuses 1 hinein, beginnen also innerhalb des von dem Gehäuse 1 umschlossenen Raumes.

Bei den in den Fig. 5 und 6 gezeigten Ausführungsbeispielen der erfindungsgemäßen Magnetvorrichtung ist keine Überwurfmutter zur Sicherung einer Lötstelle notwendig (vgl. im Gegensatz dazu die Überwurfmuttern 26 an den äußeren Stirnseiten des Gehäuses 1 bei den in den Fig. 1 und 2 dargestellten Magnetvorrichtungen aus dem Stand der Technik). Es ist nicht einmal unbedingt notwendig, daß eine erfindungsgemäße Magnetvorrichtung überhaupt ein Gehäuse aufweist. Ausführungsformen mit Gehäuse 1, wie beispielsweise in den Fig. 5 und 6 dargestellt, sind jedoch aus handhabungstechnischen Gründen bevorzugt.

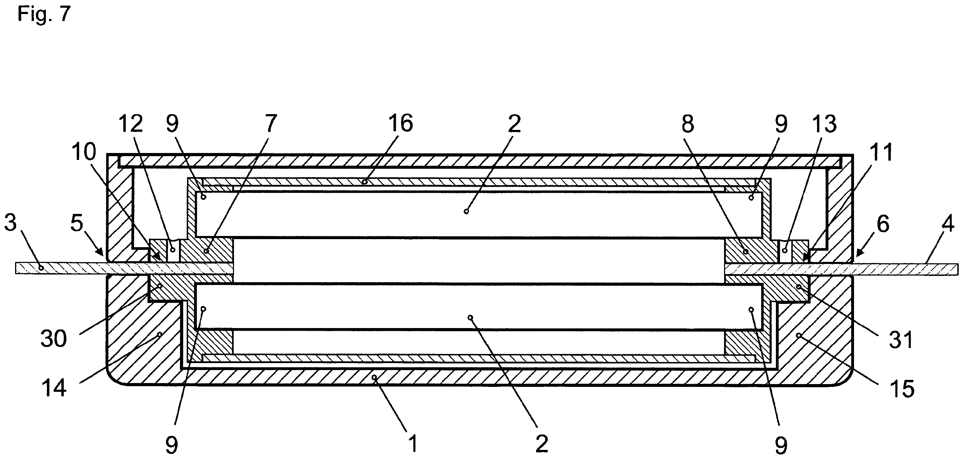

Bei den vorliegend beschriebenen Ausführungsbeispielen der erfindungsgemäßen Magnetvorrichtung werden die im Inneren des von dem Gehäuse 1 umschlossenen Raumes beginnenden Stahlseile 3 bzw. 4 jeweils durch separate Durchführungen 5 bzw. 6 (vgl. hierzu auch die Fig. 7, 10 und 12) an den jeweils einander gegenüberliegenden Stirnseiten des Gehäuses 1 aus dem vom Gehäuse 1 umschlossenen Raum hinausgeführt, wobei das erste Stahlseil 3 auf der einen Stirnseite des Gehäuses 1 aus dem Gehäuse 1 herausläuft und das zweite Stahlseil 4 auf der gegenüberliegenden Stirnseite des Gehäuses 1 aus dem Gehäuse 1 hervortritt.

Bevorzugterweise besteht jedes der beiden Stahlseile 3, 4 aus mehreren miteinander verdrillten Stahllitzen. Es können bei anderen Ausführungsbeispielen der erfindungsgemäßen Magnetvorrichtung jedoch auch andere Stahlseilausführungen zum Einsatz kommen.

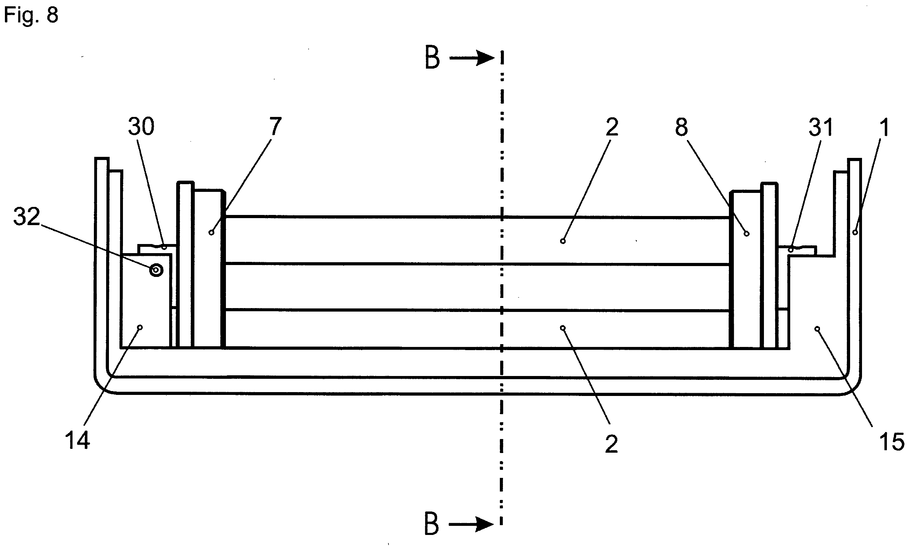

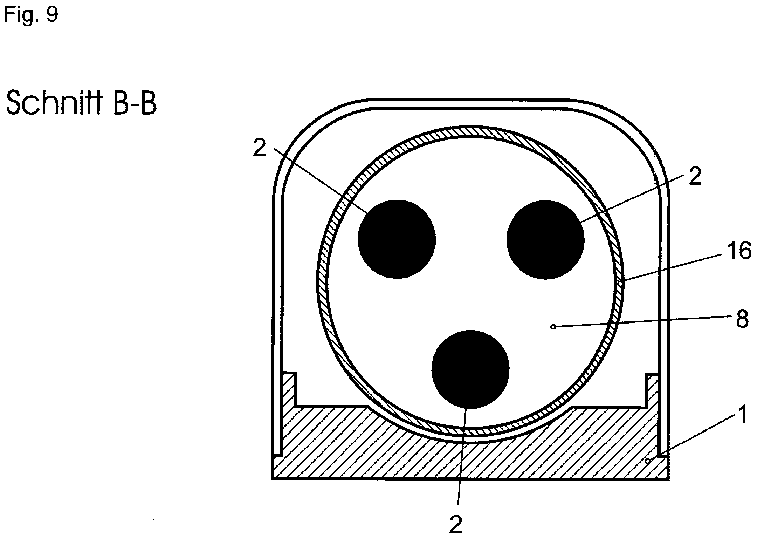

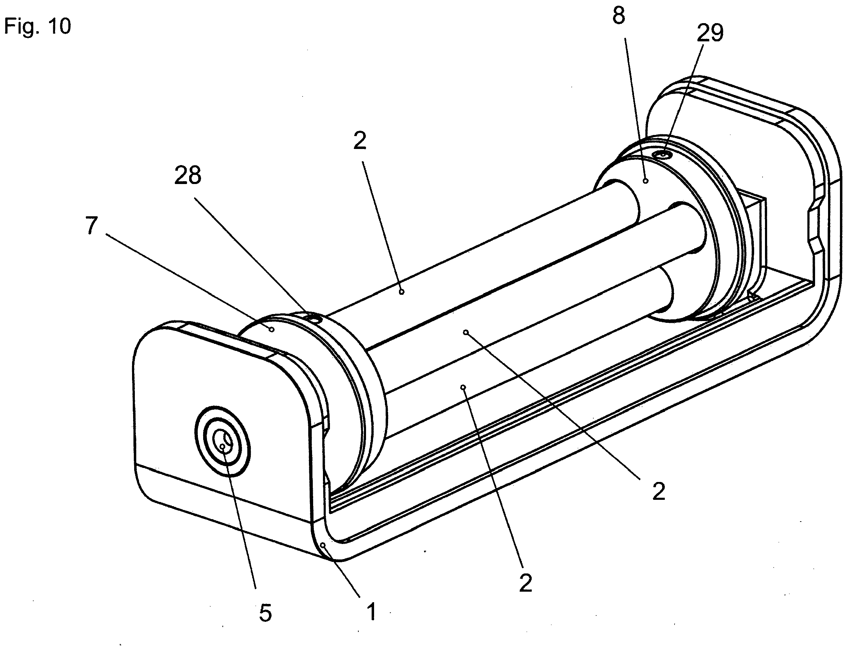

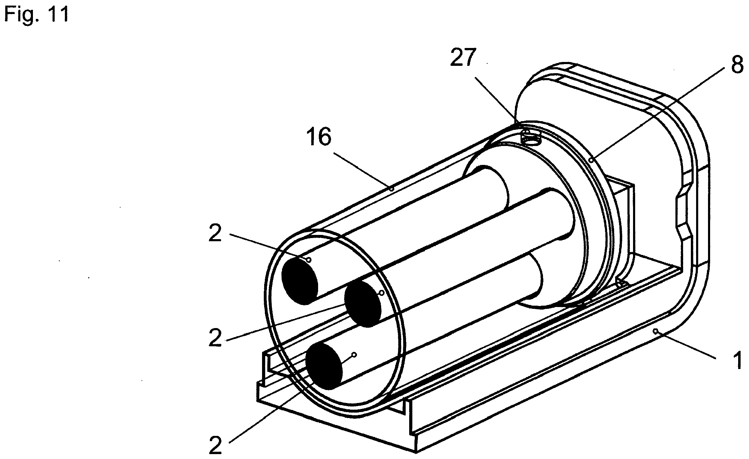

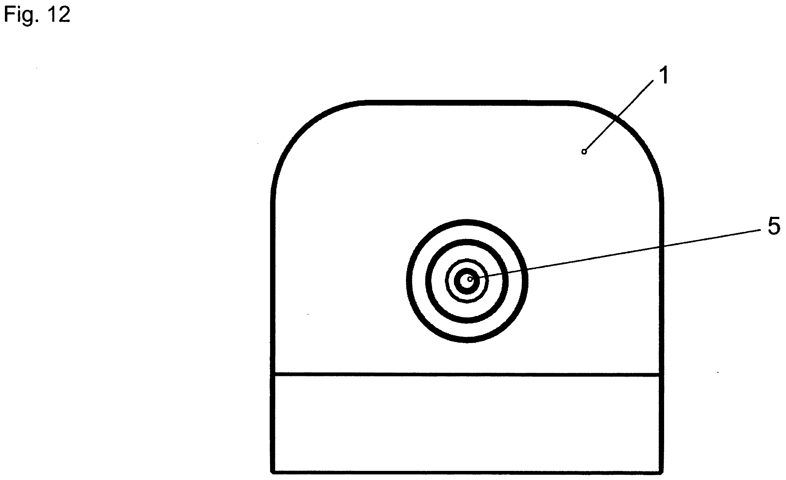

Der apparative Aufbau innerhalb des Gehäuses 1 des vorliegend beschriebenen Ausführungsbeispiels der erfindungsgemäßen Magnetvorrichtung unterscheidet sich sehr deutlich von dem apparativen Aufbau der aus dem Stand der Technik bekannten Magnetvorrichtungen. Schematische und jeweils nicht vollständige Darstellungen eines Längsschnitts durch den apparativen Aufbau innerhalb eines Gehäuses 1 eines Ausführungsbeispiels einer erfindungsgemäßen Magnetvorrichtung sind in den Fig. 7 und 8 gezeigt, während die Fig. 10 und 11 jeweils nicht vollständige und rein schematische perspektivische Ansichten des apparativen Aufbaus innerhalb des Gehäuses 1 des entsprechenden Ausführungsbeispiels der erfindungsgemäßen Magnetvorrichtung bieten. Fig. 9 ist ein um das Detail einer Röhre 16 ergänzter Querschnitt längs der Schnittlinie B - B aus Fig. 8. Fig. 12 zeigt einen Blick auf eine äußere Stirnseite des Gehäuses 1.

Genau wie bei der in Fig. 3 dargestellten Magnetvorrichtung aus dem Stand der Technik weist das in den Fig. 7 bis 12 dargestellte Ausführungsbeispiel einer erfindungsgemäßen Magnetvorrichtung drei stabförmige Permanentmagnete 2 auf, die innerhalb einer Röhre 16 (vgl. auch die Fig. 9 und 11) jeweils parallel zueinander und radial um jeweils 120° versetzt angeordnet sind. Der Übersichtlichkeit halber wurde in den rein schematischen Darstellungen von Fig. 7 und Fig. 8 einer der drei stabförmigen Permanentmagnete nicht eingezeichnet, so daß dort nur zwei stabförmige Permanentmagnete 2 zu sehen sind. In den Fig. 9 bis 11 sind die drei stabförmigen Permanentmagnete 2 jedoch alle dargestellt.

Es muß an dieser Stelle ausdrücklich betont werden, daß die Röhre 16 bei der erfindungsgemäßen Magnetvorrichtung nur optional ist. Es gibt Ausführungsbeispiele erfindungsgemäßer Magnetvorrichtungen, die gänzlich ohne eine solche Röhre auskommen, so wie es beispielsweise in Fig. 8 bzw. Fig. 10 schematisch dargestellt ist. Wie bereits weiter oben erläutert wurde, sind Ausführungsbeispiele mit der Röhre 16 jedoch bevorzugt, weil die Röhre 16, wie schon oben beschrieben, ein sehr geeignetes und nützliches Hilfsmittel bei der Montage der Magnetvorrichtung darstellt.

Alle drei stabförmigen Permanentmagnete 2 sind magnetisch gleichsinnig orientiert angeordnet, d. h. die Nordpole aller drei stabförmigen Permanentmagnete 2 weisen jeweils gemeinsam in ein und dieselbe Richtung, und die Südpole aller drei stabförmigen Permanentmagnete 2 weisen jeweils gemeinsam in die entgegengesetzte Richtung.

Das hier mit Bezug auf die Fig. 7 bis 12 näher beschriebene Ausführungsbeispiel der erfindungsgemäßen Magnetvorrichtung verwendet zwar genau drei stabförmige Permanentmagnete 2, jedoch gibt es auch andere Ausführungsbeispiele erfindungsgemäßer Magnetvorrichtungen, bei denen nicht drei stabförmige Permanentmagnete 2, sondern nur zwei oder aber sogar vier, fünf, sechs oder gar noch mehr stabförmige Permanentmagnete zum Einsatz kommen. Wesentlich ist jedoch, daß die stabförmigen Permanentmagnete immer jeweils parallel zueinander und in gleichsinniger magnetischer Orientierung gelagert sind. Die Nordpole aller dieser stabförmigen Permanentmagnete müssen also gemeinsam in ein und dieselbe Richtung weisen, so daß alle Südpole dieser stabförmigen Permanentmagnete gemeinsam in genau die entgegengesetzte Richtung zeigen.

Bei dem vorliegend unter Bezugnahme auf die Fig. 7 bis 12 näher beschriebenen Ausführungsbeispiel der erfindungsgemäßen Magnetvorrichtung sind die stabförmigen Permanentmagnete 2 alle zylinderförmig ausgebildet. Es gibt jedoch auch andere Ausführungsbeispiele erfindungsgemäßer Magnetvorrichtungen, bei denen die stabförmigen Permanentmagnete alle quaderförmig ausgebildet sind. Bei wieder anderen Ausführungsbeispielen erfindungsgemäßer Magnetvorrichtungen sind manche der stabförmigen Permanentmagnete quaderförmig und manche zylinderförmig ausgebildet. Auch der Einsatz anderer Stabformen, beispielsweise mit hexagonaler Grundfläche, ist möglich. Wichtig allein ist, daß es sich um stabförmige Permanentmagnete handelt. Aus rein produktionstechnischen Gründen werden stabförmige Permanentmagnete in Vollzylinderform bevorzugt verwendet.

Die bereits erwähnte Röhre 16 ist bei dem in den Fig. 7, 9 und 11 dargestellten erfindungsgemäßen Ausführungsbeispiel aus Aluminium gefertigt. Bei anderen Ausführungsbeispielen erfindungsgemäßer Magnetvorrichtungen besteht die Röhre 16 aus Messing oder aus einem anderen Nichteisenmetall bzw. aus einer anderen Nichteisenmetall-Legierung oder aus Kunststoff oder aus Nirosta. Darüber hinaus gibt es noch andere Ausführungsbeispiele erfindungsgemäßer Magnetvorrichtungen, bei denen eine solche Röhre überhaupt nicht vorhanden ist. Ausführungsbeispiele mit Röhre 16 sind jedoch aus dem bereits oben erläuterten Grund bevorzugt.

Bei dem vorliegend unter Bezugnahme auf die Figuren 7, 9 und 11 näher beschriebenen Ausführungsbeispiel der erfindungsgemäßen Magnetvorrichtung ist auf einer Stirnseite der Röhre 16 ein erstes Sockelelement 7 und auf der gegenüberliegenden Stirnseite der Röhre 16 ein zweites Sockelelement 8 aufgesetzt. Bei Ausführungsbeispielen der erfindungsgemäßen Magnetvorrichtung, die keine Röhre 16 aufweisen, sind die beiden Sockelelemente 7, 8 jeweils auf einander gegenüberliegenden Stirnseiten der stabförmigen Permanentmagnete 2 aufgesetzt. Man vergleiche hierzu auch die Fig. 8 und 10.

Beide Sockelelemente 7, 8 bestehen aus Stahl. Jedes der beiden Sockelelemente 7, 8 ragt bei dem vorliegend unter Bezugnahme auf die Figuren 7, 9 und 11 näher beschriebenen Ausführungsbeispiel der erfindungsgemäßen Magnetvorrichtung 15 mm in die Röhre 16 hinein und ist jeweils mittels einer in den Figuren nicht dargestellten Schraube an der Röhre 16 befestigt. In Fig. 11 ist ein zugehöriges Schraubenloch 27 in der Röhre 16 zu sehen, und die Fig. 10 zeigt entsprechende Schraubenlöcher 28 bzw. 29 in den Sockelelementen 7 bzw. 8. Anstelle dieser Befestigungsart mittels Schrauben ist die Röhre 16 bei anderen Ausführungsbeispielen der erfindungsgemäßen Magnetvorrichtung auf andere Art und Weise, beispielsweise durch Kleben oder durch Nieten, an den Sockelelementen 7, 8 fixiert. Bei wieder anderen Ausführungsbeispielen der erfindungsgemäßen Magnetvorrichtung erfolgt eine entsprechende Fixierung der Röhre 16 nur an einem der beiden Sockelelemente 7, 8. Es ist bei manchen Ausführungsbeispielen der erfindungsgemäßen Magnetvorrichtung sogar möglich, auf eine gesonderte Fixierung der Röhre 16 an den Sockelelementen 7, 8 gänzlich zu verzichten und es bei einem einfachen Aufstecken der Sockelelemente 7, 8 auf die Röhre 16 ohne weitere Sicherung bewenden zu lassen.

Bei dem vorliegend mit Bezug auf die Figuren 7 bis 12 näher beschriebenen Ausführungsbeispiel der erfindungsgemäßen Magnetvorrichtung weist jedes der beiden Sockelelemente 7, 8 auf seiner in das Innere der Röhre 16 gerichteten Seite 10 mm tiefe, becherartige Vertiefungen 9 zur paßgenauen Aufnahme der stabförmigen Permanentmagnete 2 auf, wobei für jeden stabförmigen Permanentmagneten 2 jeweils eine separate Vertiefung 9 vorgesehen ist.

Bei anderen Ausführungsbeispielen der erfindungsgemäßen Magnetvorrichtung sind die entsprechenden Vertiefungen 9 in den Sockelelementen 7, 8 flacher oder tiefer als 10 mm. Die Tiefe der Vertiefungen 9 ist dabei in geeigneter Weise auf die Länge der stabförmigen Permanentmagnete 2 abgestimmt. Die Vertiefungen 9 sollten nicht zu flach sein, damit sowohl eine gute Konzentration des magnetischen Flusses als auch eine sichere mechanische Lagerung der stabförmigen Permanentmagnete 2 sichergestellt ist. Die Vertiefungen 9 sollten aber auch nicht zu tief sein, weil sonst die Gefahr magnetischer Kurzschlüsse besteht.

Es gibt Ausführungsbeispiele der erfindungsgemäßen Magnetvorrichtung, bei denen jede Vertiefung 9 mindestens 4 mm oder mindestens 5 mm oder mindestens 6 mm oder mindestens 7 mm oder mindestens 8 mm oder mindestens 9 mm oder mindestens 10 mm oder mindestens 12 mm oder mindestens 14 mm oder mindestens 16 mm oder mindestens 18 mm oder mindestens 20 mm tief ist. Ferner gibt es Ausführungsbeispiele der erfindungsgemäßen Magnetvorrichtung, bei denen jede Vertiefung 9 höchstens 50 mm oder höchstens 45 mm oder höchstens 40 mm oder höchstens 35 mm oder höchstens 30 mm tief ist. Dabei ist es nicht unbedingt notwendig, daß alle Vertiefungen 9 gleich tief sind. Aus produktionstechnischen Gründen ist es jedoch sinnvoll und daher bevorzugt, innerhalb einer erfindungsgemäßen Magnetvorrichtung alle Vertiefungen 9 gleich tief und alle stabförmigen Permanentmagnete 2 gleich lang auszuführen. Bei dem in den Figuren 7 bis 12 schematisch dargestellten speziellen Ausführungsbeispiel der erfindungsgemäßen Magnetvorrichtung beispielsweise hat jeder einzelne der drei stabförmigen Permanentmagnete 2 eine Länge von 150 mm, während jede einzelne der Vertiefungen 9, wie bereits oben erwähnt, 10 mm tief ist. Bei anderen Ausführungsbeispielen der erfindungsgemäßen Magnetvorrichtung sind die stabförmigen Permanentmagnete 2 länger oder kürzer als 150 mm.

Bei dem vorliegend unter Bezugnahme auf die Figuren 7 bis 12 dargestellten Ausführungsbeispiel der erfindungsgemäßen Magnetvorrichtung sind die Vertiefungen 9 und die jeweils in ihnen steckenden stabförmigen Permanentmagnete 2 derart paßgenau aufeinander abgestimmt, daß das Spiel maximal jeweils 0,5 mm beträgt. Bei anderen Ausführungsbeispielen der erfindungsgemäßen Magnetvorrichtung ist jede Vertiefung 9 sogar derart paßgenau auf den in ihr steckenden stabförmigen Permanentmagneten 2 abgestimmt, daß das Spiel maximal 0,2 mm beträgt.

Bei den in den Fig. 5 bis 12 dargestellten Ausführungsbeispielen der erfindungsgemäßen Magnetvorrichtung weist das erste Sockelelement 7 eine von seiner Permanentmagnet-Aufnahmeseite abgewandten Seite ausgehende Bohrung oder Durchbohrung 10 zur Aufnahme eines Endabschnittes des ersten Stahlseils 3 auf. Bei den in den Fig. 5 bis 12 dargestellten Ausführungsbeispielen der erfindungsgemäßen Magnetvorrichtung weist das zweite Sockelelement 8 eine von seiner Permanentmagnet-Aufnahmeseite abgewandten Seite ausgehende Bohrung oder Durchbohrung 11 zur Aufnahme eines Endabschnittes des zweiten Stahlseils 4 auf. Bei dem in Fig. 7 dargestellten Ausführungsbeispiel der erfindungsgemäßen Magnetvorrichtung sind die eben genannten Bohrungen 10, 11 tatsächlich durch das jeweilige Sockelelement 7, 8 vollständig hindurchgehende Bohrungen, also Durchbohrungen. Bei anderen Ausführungsbeispielen der erfindungsgemäßen Magnetvorrichtung gehen diese Bohrungen nicht vollständig durch das jeweilige Sockelelement 7, 8 hindurch, sind also keine Durchbohrungen.

In der Bohrung oder Durchbohrung 10 des ersten Sockelelements 7 steckt ein Endabschnitt des ersten Stahlseils 3. In der Bohrung oder Durchbohrung 11 des zweiten Sockelelements 8 steckt ein Endabschnitt des zweiten Stahlseils 4. Dies ist in Fig. 7 schematisch dargestellt.

Bei dem vorliegend unter Bezugnahme auf die Fig. 7 bis 12 näher beschriebenen Ausführungsbeispiel der erfindungsgemäßen Magnetvorrichtung hat das erste Sockelelement 7 eine runde Peripherie und ist so ausgebildet, daß es auf der von seiner Permanentmagnet-Aufnahmeseite abgewandten Seite einen ersten Fortsatz 30 aufweist, dessen Durchmesser kleiner ist als der Durchmesser des ersten Sockelelements 7 an seiner weitesten Stelle (vgl. hierzu insbesondere die Fig. 7 und 8). Das zweite Sockelelement 8 ist bei dem vorliegend näher betrachteten Ausführungsbeispiel der erfindungsgemäßen Magnetvorrichtung ganz analog dem ersten Sockelelement 7 aufgebaut. Dementsprechend hat das zweite Sockelelement 8 auch eine runde Peripherie und ist so ausgebildet, daß es auf der von seiner Permanentmagnet-Aufnahmeseite abgewandten Seite einen zweiten Fortsatz 31 aufweist, dessen Durchmesser kleiner ist als der Durchmesser des zweiten Sockelelements 8 an seiner weitesten Stelle. Die Durchmesser der beiden Fortsätze 30, 31 sind bei dem hier betrachteten speziellen Ausführungsbeispiel nur ungefähr halb so groß wie die Durchmesser der jeweiligen Sockelelemente 7, 8 an ihren jeweils weitesten Stellen (siehe Fig. 7 und 8).

Eine derartige Formgebung ist zwar bevorzugt, jedoch gibt es auch andere Ausführungsbeispiele der erfindungsgemäßen Magnetvorrichtung, bei denen die Durchmesser oder Breiten der Fortsätze 30, 31 nur sehr geringfügig kleiner sind als die Durchmesser der jeweiligen Sockelelemente 7, 8 an ihren jeweils weitesten Stellen, oder bei denen sogar zwischen beiden genannten Durchmessern überhaupt kein Unterschied besteht.

Bei jedem Ausführungsbeispiel der erfindungsgemäßen Magnetvorrichtung, bei dem ein erster Fortsatz 30 und/oder ein zweiter Fortsatz 31 vorhanden ist, gilt jedoch folgendes: Der erste Fortsatz 30 ist integraler Bestandteil des ersten Sockelelements 7, so daß das erste Sockelelement 7 samt seinem ersten Fortsatz 30 ein einteiliges Stück ist. Der zweite Fortsatz 31 ist integraler Bestandteil des zweiten Sockelelements 8, so daß das zweite Sockelelement 8 samt seinem zweiten Fortsatz 31 ein einteiliges Stück ist.

Bei dem in den Figuren 5 bis 12 dargestellten Ausführungsbeispiel der erfindungsgemäßen Magnetvorrichtung ist jeder der beiden Fortsätze 30, 31 mit jeweils einem Schraubenloch 12, 13 versehen, durch das eine Schraube zum Fixieren des jeweiligen Stahlseils 3, 4 geführt ist (vgl. diesbezüglich Fig. 7).

Bei anderen Ausführungsbeispielen der erfindungsgemäßen Magnetvorrichtung kann das Fixieren des ersten Stahlseils 3 in dem ersten Sockelelement 7 und/oder das Fixieren des zweiten Stahlseils 4 in dem zweiten Sockelelement 8 auch auf andere Art und Weise, beispielsweise durch Verlöten, Verpressen oder mittels Spannkeils erfolgen.

Abgesehen von der vorstehend genannten Schraube zum Fixieren des jeweiligen Stahlseils 3, 4 und abgesehen von der weiter vorstehend genannten Schraube zum Fixieren der Röhre 16 ist jedes der beiden stählernen Sockelelemente 7, 8 einschließlich des jeweils zugehörigen Fortsatzes 30, 31 einstückig ausgebildet und eignet sich daher besser zum Konzentrieren des magnetischen Flusses in die Stahlseile 3, 4 als die aus mehreren Einzelteilen zusammengesetzte entsprechende Anschlußstelle bei der Magnetvorrichtung aus dem Stand der Technik. Darüber hinaus gewährleisten die 10 mm tiefen becherartigen Vertiefungen 9 zur paßgenauen Aufnahme der stabförmigen Permanentmagnete 2 bei dem beschriebenen Ausführungsbeispiel der erfindungsgemäßen Magnetvorrichtung einen weit innigeren Kontakt zwischen den stabförmigen Permanentmagneten 2 und dem jeweiligen Sockelelement 7, 8, als es bei der Konstruktion mit den Stahlscheiben 21, 24 aus dem Stand der Technik möglich war (vgl. zum Stand der Technik die Fig. 3 und 4). Alle diese Faktoren zusammengenommen führen zu einem gegenüber der Magnetvorrichtung aus dem Stand der Technik optimierten magnetischen Fluß in die Stahlseile 3, 4, woraus eine Optimierung des magnetischen Kreises der Magnetvorrichtung insgesamt resultiert. Dies ist für alle Anwendungsfälle der erfindungsgemäßen Magnetvorrichtung vorteilhaft. Ganz besonders vorteilhaft ist es insbesondere für jene Anwendungsfälle, bei denen der menschliche Körper in unmittelbaren Kontakt mit den genannten Griffelementen 17, 18 gebracht und auf diese Weise direkt in den magnetischen Kreis der Magnetvorrichtung eingeschlossen wird.

Bei dem in den Fig. 7 bis 12 dargestellten Ausführungsbeispiel der erfindungsgemäßen Magnetvorrichtung ist die Durchbohrung 10 bzw. 11 zur Aufnahme eines Endabschnittes des ersten Stahlseils 3 bzw. eines Endabschnittes des zweiten Stahlseils 4 jeweils im Zentrum des jeweiligen Sockelelementes 7, 8 angeordnet, und alle stabförmigen Permanentmagnete 2 befinden sich, jeweils um 120° versetzt, radial im gleichen Abstand zum Zentrum des jeweiligen Sockelelements 7, 8. Eine derartige zentrierte Anordnungsweise mit hoher Symmetrie ist im Hinblick auf die Optimierung des in die Stahlseile 3, 4 gerichteten Magnetflusses ganz besonders vorteilhaft.

Das Gehäuse 1 des in den Fig. 7 bis 12 dargestellten Ausführungsbeispiels der erfindungsgemäßen Magnetvorrichtung hat in seinem Inneren einen ersten Lagerblock 14, auf dem das erste Sockelelement 7 fixiert ist. Konkret liegt der erste Fortsatz 30 auf dem ersten Lagerblock 14 auf und wird dort mittels einer Schraube festgehalten. Ein entsprechendes Schraubenloch 32 ist in Fig. 8 zu sehen.

Ganz analog dazu befindet sich ein zweiter Lagerblock 15 innerhalb des Gehäuses 1, und auf diesem zweiten Lagerblock 15 ist das zweite Sockelelement 8 fixiert. Konkret liegt der zweite Fortsatz 31 auf dem zweiten Lagerblock 15 auf und wird dort mittels einer Schraube festgehalten.

Obwohl bei dem hier unter Bezugnahme auf die Figuren 5 bis 12 beschriebenen Ausführungsbeispiel der erfindungsgemäßen Magnetvorrichtung die Fixierung der Sockelelemente 7, 8 mit ihren Fortsätzen 30, 31 auf den Lagerblöcken 14, 15 jeweils mittels einer Schraubverbindung erfolgt, können bei anderen Ausführungsbeispielen der erfindungsgemäßen Magnetvorrichtung die Sockelelemente 7, 8 auch auf andere Art und Weise, beispielsweise durch Kleben oder durch Verkeilen oder durch Verklemmen, an den Lagerblöcken 14, 15 fixiert sein.

Bei einigen Ausführungsbeispielen der erfindungsgemäßen Magnetvorrichtung haben die freien Stirnseiten, d.h. die vom jeweiligen Stahlseil 3, 4 abgewandten Stirnseiten der beiden Griffelemente 17, 18 eine speziell ausgebildete Form. Das erste Griffelement 17 ist an seiner freien Stirnseite konvex ausgebildet, und das zweite Griffelement 18 ist an seiner freien Stirnseite konkav ausgebildet. Dabei ist die Form der beiden genannten freien Stirnseiten derart aufeinander abgestimmt, daß sie spaltfrei aneinander anliegen können. Die Permanentmagnete innerhalb der beiden Griffelemente 17, 18 sind derart angeordnet, daß der an der konvex ausgebildeten Stirnseite befindliche Magnetpol gegensinnig zu dem an der konkav ausgebildeten Stirnseite befindlichen Magnetpol ist, so daß die Stirnseiten der beiden Griffelemente 17, 18 magnetisch aneinander haften, wenn sie zusammengefügt werden. Eine solche Konstellation findet beispielsweise Anwendung, wenn mit der Magnetvorrichtung auf einen Wohnraum eingewirkt oder - bei entsprechender Verlängerung der Stahlseile - ein ganzes Haus umfaßt werden soll. Sicherheitshalber können die beiden Griffelemente 17, 18 dann auch noch wegen etwaig sporadisch auftretender Zugbelastung mit einer Sicherungseinrichtung 19, beispielsweise einem Klebeband, aneinander fixiert werden, so wie es in Fig. 6 schematisch gezeigt ist.

Die im vorstehenden Absatz beschriebene besondere Gestaltung der freien Stirnseiten der beiden Griffelemente 17, 18 hat den ganz speziellen Vorteil, daß sehbehinderte Personen die magnetische Polausrichtung der Griffelemente auf einfache Weise ertasten können. Für manche therapeutische Anwendungen ist es nämlich von Bedeutung, in welcher absoluten magnetischen Orientierung der menschliche Körper in den magnetischen Fluß der Magnetvorrichtung eingeschaltet wird.

Entsprechende Griffelemente 17, 18 und/oder die vorstehend erläuterte Sicherungseinrichtung 19 können auch bei Stahlseilen 3, 4 in Ausführungsbeispielen gemäß der zweiten Beispielvariante der erfindungsgemäßen Magnetvorrichtung vorhanden sein, welche nachfolgend mit Bezug auf die Fig. 13 näher erläutert werden.

Fig. 13 zeigt schematisch einen Längsschnitt durch das Gehäuse 1 eines Ausführungsbeispiels gemäß der zweiten Beispielvariante der erfindungsgemäßen Magnetvorrichtung.

Der Unterschied zwischen der oben beschriebenen ersten Beispielvariante der erfindungsgemäßen Magnetvorrichtung und der nun zu diskutierenden zweiten Beispielvariante der erfindungsgemäßen Magnetvorrichtung besteht in der Art und Weise, wie der Kontakt zwischen dem Stahlseil bzw. den Stahlseilen 3, 4 einerseits und den Sockelelementen 7, 8 andererseits hergestellt wird, was sich dementsprechend insbesondere auf die Gestaltung der Sockelelemente 7, 8 auswirkt. Nicht betroffen sind jedoch die Vertiefungen 9 in den Sockelelementen 7, 8 sowie die Anzahl, Form und Anordnungsweise der in diesen Vertiefungen 9 steckenden stabförmigen Permanentmagnete 2, ferner die etwaig vorhandenen integralen Fortsätze 30, 31 der Sockelelemente 7, 8, das etwaig vorhandene Gehäuse 1, die etwaig vorhandene Röhre 16, die etwaig vorhandenen Lagerblöcke 14, 15 sowie die Ausführungsweise der Stahlseile 3, 4 als solche. Insoweit gilt alles, was oben für die erste Beispielvariante der erfindungsgemäßen Magnetvorrichtung gesagt wurde, in gleichem Maße auch für die zweite Beispielvariante der erfindungsgemäßen Magnetvorrichtung. Auch bei der zweiten Beispielvariante der erfindungsgemäßen Magnetvorrichtung sind beide Sockelelemente 7, 8 - ggf. samt ihrem jeweiligen Fortsatz 30, 31 - jeweils einteilig aus Stahl gefertigt. Man vergleiche insoweit auch die Darstellung der Fig. 13, welche ein Ausführungsbeispiel für die zweite Beispielvariante der erfindungsgemäßen Magnetvorrichtung zeigt, mit der Darstellung von Fig. 7, welche ein Ausführungsbeispiel für die erste Beispielvariante der erfindungsgemäßen Magnetvorrichtung zeigt.

Im Unterschied zu dem in Fig. 7 dargestellten Ausführungsbeispiel der ersten Beispielvariante der erfindungsgemäßen Magnetvorrichtung ist gemäß dem in Fig. 13 gezeigten Ausführungsbeispiel der zweiten Beispielvariante der erfindungsgemäßen Magnetvorrichtung ein Ende des ersten Stahlseils 3 in einem ersten magnetisch leitenden Zylinder 33 befestigt, welcher so ausgebildet ist, daß er in jedem Falle an dem ersten Sockelelement 7 lösbar befestigbar ist, indem er durch die magnetische Kraft der stabförmigen Permanentmagnete 2 auf das erste Sockelelement 7 gezogen wird. Ferner ist gemäß dem in Fig. 13 dargestellten Ausführungsbeispiel der zweiten Beispielvariante der erfindungsgemäßen Magnetvorrichtung ein Ende des zweiten Stahlseils 4 in einem zweiten magnetisch leitenden Zylinder 34 befestigt, welcher so ausgebildet ist, daß er in jedem Falle an dem zweiten Sockelelement 8 lösbar befestigbar ist, indem er durch die magnetische Kraft der stabförmigen Permanentmagnete 2 auf das zweite Sockelelement 8 gezogen wird.

Gemäß einem anderen Ausführungsbeispiel der zweiten Beispielvariante der erfindungsgemäßen Magnetvorrichtung ist nur ein einziges Stahlseil, nämlich das erste Stahlseil 3, vorhanden. Dieses erste Stahlseil 3 ist bei diesem anderen Ausführungsbeispiel mit seinem ersten Ende in einem ersten magnetisch leitenden Zylinder 33 befestigt, welcher so ausgebildet ist, daß er in jedem Falle an dem ersten Sockelelement 7 lösbar befestigbar ist, indem er durch die magnetische Kraft der stabförmigen Permanentmagnete 2 auf das erste Sockelelement 7 gezogen wird. Mit seinem zweiten Ende ist das erste Stahlseil 3 bei diesem anderen Ausführungsbeispiel der zweiten Beispielvariante der erfindungsgemäßen Magnetvorrichtung in einem zweiten magnetisch leitenden Zylinder 34 befestigt, welcher so ausgebildet ist, daß er in jedem Falle an dem zweiten Sockelelement 8 lösbar befestigbar ist, indem er durch die magnetische Kraft der stabförmigen Permanentmagnete 2 auf das zweite Sockelelement 8 gezogen wird. Dieses eben beschriebene andere Ausführungsbeispiel der zweiten Beispielvariante der. erfindungsgemäßen Magnetvorrichtung wird im Grunde genommen auch von der Fig. 13 verdeutlicht. Man muß zu diesem Zweck in Fig. 13 lediglich gedanklich das Bezugszeichen "4" durch das Bezugszeichen "3" ersetzen.

. Bei den beiden vorstehend unter Bezugnahme auf die Fig. 13 näher erläuterten Ausführungsbeispielen der zweiten Beispielvariante der erfindungsgemäßen Magnetvorrichtung ist im ersten Sockelelement 7 eine Bohrung oder Durchbohrung zur Aufnahme eines Endabschnittes eines Stahlseils nicht notwendig, und auch im zweiten Sockelelement 8 ist eine solche Bohrung oder Durchbohrung zur Aufnahme eines Endabschnittes eines Stahlseils nicht notwendig. Derartige vorstehend genannte Bohrungen oder Durchbohrungen können aber durchaus auch bei jenen vorstehend näher erläuterten Ausführungsbeispielen der zweiten Beispielvariante der erfindungsgemäßen Magnetvorrichtung vorhanden sein und sind aus rein produktionspraktischen Gründen tatsächlich dann vorhanden, wenn man zunächst die Sockelelemente 7, 8 als solche in größeren Stückzahlen automatisiert und einheitlich produziert, sie anschließend aber in durchaus unterschiedlichen Ausführungsbeispielen sowohl für die erste als auch für die zweite Beispielvariante der erfindungsgemäßen Magnetvorrichtung einsetzt. Die dementsprechend in Fig. 13 im ersten Sockelelement 7 bzw. im zweiten Sockelelement 8 angedeuteten und nicht mit Bezugszeichen versehenen Sacklochbohrungen in der jeweils von der Aufnahmeseite für die stabförmigen Permanentmagnete 2 abgewandten Seite des jeweiligen Sockelelements 7, 8 haben jedenfalls im Rahmen genau der oben mit Bezug auf Fig. 13 näher beschriebenen beiden Ausführungsbeispiele der zweiten Beispielvariante der erfindungsgemäßen Magnetvorrichtung keine funktionelle Bedeutung. Es gibt jedoch auch andere Ausführungsbeispiele der zweiten Beispielvariante der erfindungsgemäßen Magnetvorrichtung, bei denen derartige Sacklochbohrungen in den Sockelelementen 7, 8 jeweils groß genug und die magnetisch leitenden Zylinder 33, 34 jeweils klein genug ausgeführt sind, so daß die Sacklochbohrungen in den Sockelelementen 7, 8 zur Aufnahme der magnetisch leitenden Zylinder 33, 34 samt dem bzw. den darin befestigten Stahlseil(en) 3 bzw. 3, 4 dienen können. Sogar solche Ausführungsbeispiele der zweiten Beispielvariante der erfindungsgemäßen Magnetvorrichtung gibt es, bei denen die magnetisch leitenden Zylinder 33, 34 gänzlich fehlen und nur das bzw. die Stahlseil(e) 3 bzw. 3, 4 lösbar befestigbar in den vorstehend genannten Sacklochbohrungen der Sockelelemente 7, 8 andockt bzw. andocken, indem es bzw. sie einfach durch die magnetische Kraft der stabförmigen Permanentmagnete 2 in die jeweiligen Sacklochbohrungen hineingezogen wird bzw. hineingezogen werden. Bevorzugt sind jedoch solche Ausführungsbeispiele der zweiten Beispielvariante der erfindungsgemäßen Magnetvorrichtung, bei denen die genannten magnetisch leitenden Zylinder 33, 34 vorhanden sind und jeweils mit ihren Stirnseiten an jeweils eine Stirnseite eines Sockelelementes 7, 8 magnetisch andocken, so wie es in Fig. 13 gezeigt ist.

Auch bei der zweiten Beispielvariante der erfindungsgemäßen Magnetvorrichtung gibt es, genau wie bei der ersten Beispielvariante der erfindungsgemäßen Magnetvorrichtung, Ausführungsbeispiele mit Gehäuse 1 zur Aufnahme der stabförmigen Permanentmagnete 2 und zur Aufnahme der beiden Sockelelemente 7, 8. Genau wie bei der ersten Beispielvariante der erfindungsgemäßen Magnetvorrichtung gibt es jedoch auch bei der zweiten Beispielvariante der erfindungsgemäßen Magnetvorrichtung andere Ausführungsbeispiele, bei denen ein solches Gehäuse nicht vorhanden ist.

Falls ein solches Gehäuse 1 vorhanden ist, so ist es bevorzugterweise aus einem Nichteisenmetall gefertigt. Ganz besonders bevorzugt wird hierfür Aluminium verwendet. Es gibt aber auch andere Ausführungsbeispiele der erfindungsgemäßen Magnetvorrichtung, bei denen das Gehäuse 1 beispielsweise aus Kunststoff oder aus einer Nichteisenmetall-Legierung oder allgemein aus einer Metall-Legierung oder aus Eisenmetall oder aus Stahl gefertigt ist.

Sofern ein zur zweiten Beispielvariante der erfindungsgemäßen Magnetvorrichtung gehörendes Ausführungsbeispiel der erfindungsgemäßen Magnetvorrichtung mit einem Gehäuse 1 versehen ist, so wie es auch in Fig. 13 gezeigt wird, hat dieses Gehäuse 1 auf einer seiner beiden Stirnseiten eine erste Öffnung zum Hineinstecken eines Endabschnittes des ersten Stahlseils 3 und auf einer anderen seiner beiden Stirnseiten eine zweite Öffnung zum Hineinstecken eines Endabschnittes eines zweiten Stahlseils 4 oder zum Hineinstecken eines anderen Endabschnittes des ersten Stahlseils 3, wobei die genannte erste Öffnung des Gehäuses 1 dem ersten Sockelelement 7 stirnseitig gegenüberliegt und wobei die genannte zweite Öffnung des Gehäuses 1 dem zweiten Sockelelement 8 stirnseitig gegenüberliegt. Bevorzugterweise wird dabei eine solche Anordnung gewählt, daß die genannte erste Öffnung des Gehäuses 1 dem ersten Sockelelement 7 axial zentriert gegenüberliegt und die genannte zweite Öffnung des Gehäuses 1 dem zweiten Sockelelement 8 axial zentriert gegenüberliegt. Eine derartige zentrierte Anordnungsweise ist im Hinblick auf die Optimierung des Magnetflusses ganz besonders vorteilhaft.

Sind bei einem zur zweiten Beispielvariante der erfindungsgemäßen Magnetvorrichtung gehörenden Ausführungsbeispiel der erfindungsgemäßen Magnetvorrichtung mit Gehäuse 1 außerdem der erste magnetisch leitende Zylinder 33 und der zweite magnetisch leitende Zylinder 34 vorhanden, so ist der erste magnetisch leitende Zylinder 33 derart ausgebildet, daß er durch die genannte erste Öffnung in dem Gehäuse 1 hindurch gesteckt werden kann, und der zweite magnetisch leitende Zylinder 34 ist derart ausgebildet, daß er durch die genannte zweite Öffnung in dem Gehäuse 1 hindurch gesteckt werden kann, so wie es in Fig. 13 gezeigt ist.

In jedem Falle besteht der besondere Vorteil aller zur zweiten Beispielvariante der erfindungsgemäßen Magnetvorrichtung gehörenden Ausführungsbeispiele der erfindungsgemäßen Magnetvorrichtung in ihrer ganz besonders hohen Einsatz- und Anwendungsflexibilität. Da bei diesen Ausführungsbeispielen das erste Stahlseil 3 bzw. das erste Stahlseil 3 und das zweite Stahlseil 4 je nach gewünschtem Einsatzzweck einfach aufgesteckt und auch einfach wieder abgezogen werden kann bzw. können, ist eine Anpassung des jeweiligen Ausführungsbeispiels der erfindungsgemäßen Magnetvorrichtung an wechselnde Einsatzbedingungen schnell und problemlos möglich. So kann ein Anwender beispielsweise ein solches Ausführungsbeispiel der erfindungsgemäßen Magnetvorrichtung hauptsächlich die meiste Zeit ohne aufgestecktes Stahlseil 3, 4 in stationärer Weise zur Raumharmonisierung seines Wohnraumes verwenden, jedoch sporadisch wünschen, sich selbst unmittelbar in den magnetischen Kreis der Magnetvorrichtung zwecks individueller Behandlung einschalten zu wollen. In letzterem Falle braucht der Anwender dann nur in ganz einfacher und unkomplizierter Weise das erste Stahlseil 3 und das zweite Stahlseil 4 wie oben beschrieben mit ihrem jeweils einen Ende an das erste Sockelelement 7 bzw. an das zweite Sockelelement 8 anzustecken und die jeweils anderen, ggf. mit den oben beschriebenen Griffelementen 17, 18 versehenen Enden der beiden Stahlseile 3, 4 in jeweils einer Hand festzuhalten. Sobald der Anwender nach seinen subjektiven Wünschen genügend lange diese individuelle Behandlung vollzogen hat, kann er wieder ganz einfach zur stationären Raumharmonisierung übergehen, indem er beide Stahlseile 3, 4 wieder von den jeweiligen Sockelelementen 7, 8 abzieht. Zu diesem Zweck muß zwar vom Anwender etwas Muskelkraft aufgewendet werden, um die jeweilige magnetische Anziehungskraft zu überwinden, jedoch ist dies eine nicht übermäßige Kraftanstrengung, die grundsätzlich von Menschen aller hier als Anwender relevanten Altersgruppen aufgebracht werden kann.

Im übrigen läßt sich die Magnetvorrichtung auch bequemer von einem Einsatzort zu einem anderen Einsatzort transportieren, wenn die jeweiligen Stahlseile 3, 4 von den jeweiligen Sockelelementen 7, 8 abgezogen wurden. Die zentrale Einheit mit den stabförmigen Permanentmagneten 2 und den Sockelelementen 7, 8 einerseits und das Stahlseil 3 oder die Stahlseile 3, 4 andererseits lassen sich bequemer verstauen oder tragen, wenn sie voneinander separiert sind.

Ferner sei der Vollständigkeit halber noch erwähnt, daß es, ähnlich wie bei den Magnetvorrichtungen aus dem Stand der Technik, auch bei Ausführungsbeispielen der oben genannten ersten Beispielvariante der erfindungsgemäßen Magnetvorrichtung und bei Ausführungsbeispielen der oben genannten zweiten Beispielvariante der erfindungsgemäßen Magnetvorrichtung möglich ist, zwei, drei oder noch mehr dieser Magnetvorrichtungen in Reihe zusammenzuschalten. In einem solchen Falle verbindet dann jeweils ein Stahlseil ein zweites Sockelelement 8 einer ersten zentralen Einheit, welche stabförmige Permanentmagnete 2 aufweist, mit dem ersten Sockelelement 7 einer weiteren zentralen Einheit, welche weitere stabförmige Permanentmagnete 2 aufweist. Dementsprechend kann dann ferner ein nächstes Stahlseil vorgesehen sein, welches das zweite Sockelelement 8 der vorstehend genannten weiteren zentralen Einheit mit dem ersten Sockelelement 7 einer dritten zentralen Einheit verbindet und immer so fort, bis der Kreis irgendwann geschlossen wird oder bis doch einmal jeweils ein Ende eines Stahlseils in ein erstes Griffelement 17 und ein Ende eines anderen Stahlseils in ein zweites Griffelement 18 mündet. Dabei können entweder ausschließlich Ausführungsbeispiele der ersten Beispielvariante der erfindungsgemäßen Magnetvorrichtung oder ausschließlich Ausführungsbeispiele der zweiten Beispielvariante der erfindungsgemäßen Magnetvorrichtung verwendet werden. Es können dabei aber sogar auch in beliebiger Weise Ausführungsbeispiele der ersten Beispielvariante der erfindungsgemäßen Magnetvorrichtung und Ausführungsbeispiele der zweiten Beispielvariante der erfindungsgemäßen Magnetvorrichtung miteinander gemischt gekoppelt werden. Bevorzugt verwendet man solche Reihenschaltungen erfindungsgemäßer Magnetvorrichtungen, um ein ganzes Haus zu umfassen.

Die vorliegende Beschreibung abschließend seien nachfolgend noch einmal alle im vorstehenden Beschreibungstext verwendeten Bezugszeichen zusammenfassend aufgelistet:
- 1: - Gehäuse
- 2: - stabförmiger Permanentmagnet
- 3: - erstes Stahlseil
- 4: - zweites Stahlseil
- 5: - erste Durchführung
- 6: - zweite Durchführung
- 7: - erstes Sockelelement
- 8: - zweites Sockelelement
- 9: - Vertiefung im ersten bzw. zweiten Sockelelement
- 10: - Bohrung oder Durchbohrung im ersten Sockelelement
- 11: - Bohrung oder Durchbohrung im zweiten Sockelelement
- 12: - Schraubenloch im ersten Sockelelement für eine Schraube zum Fixieren des ersten Stahlseils
- 13: - Schraubenloch im zweiten Sockelelement für eine Schraube zum Fixieren des zweiten Stahlseils
- 14: - erster Lagerblock
- 15: - zweiter Lagerblock
- 16: - Röhre
- 17: - erstes Griffelement
- 18: - zweites Griffelement
- 19: - Sicherungseinrichtung
- 20: - Stahlröhre
- 21: - erste Stahlscheibe
- 22: - Loch in der ersten Stahlscheibe zum Hindurchstecken eines stabförmigen Permanentmagneten
- 23: - Schraube
- 24: - zweite Stahlscheibe
- 25: - Kontermuttern
- 26: - Überwurfmutter
- 27: - Schraubenloch in der Röhre
- 28: - Schraubenloch im ersten Sockelelement für eine Schraube zum Fixieren der Röhre
- 29: - Schraubenloch im zweiten Sockelelement für eine Schraube zum Fixieren der Röhre
- 30: - erster Fortsatz
- 31: - zweiter Fortsatz
- 32: - Schraubenloch im ersten Lagerblock
- 33: - erster magnetisch leitender Zylinder
- 34: - zweiter magnetisch leitender Zylinder

## Patentansprüche

1. Magnetvorrichtung mit zwei oder mit drei oder mit vier oder mit fünf oder mit sechs oder mit mehr als sechs stabförmigen Permanentmagneten (2), die alle zueinander jeweils parallel und magnetisch alle gleichsinnig orientiert angeordnet sind, wobei die Magnetvorrichtung die folgende Merkmale aufweist:
- ein erstes einteiliges Sockelelement (7) aus Stahl und ein zweites einteiliges Sockelelement (8) aus Stahl, die beide jeweils Vertiefungen (9) zur Aufnahme von Stirnseiten der stabförmigen Permanentmagnete (2) aufweisen, wobei in jedem der beiden Sockelelemente (7, 8) für jeden einzelnen der stabförmigen Permanentmagnete (2) jeweils eine separate Vertiefung (9) vorgesehen ist und die Permanentmagnete (2) in den beiden Sockelelementen (7, 8) derart gelagert sind, daß alle Permanentmagnete (2) mit ihrer jeweiligen Nordpolseite in dem ersten Sockelelement (7) und mit ihrer jeweiligen Südpolseite in dem zweiten Sockelelement (8) stecken,
- ein erstes Stahlseil (3), mit einem ersten Ende und einem zweiten Ende, wobei das erste Ende des ersten Stahlseils (3) in einem ersten magnetisch leitenden Zylinder (33) befestigt ist, welcher so ausgebildet ist, daß er an dem ersten Sockelelement (7) lösbar befestigbar ist, indem er durch die magnetische Kraft der stabförmigen Permanentmagnete (2) auf das erste Sockelelement (7) gezogen wird, und
- ein zweites Stahlseil (4) mit einem ersten Ende und einem zweiten Ende, wobei das erste Ende des zweiten Stahlseils (4) in einem zweiten magnetisch leitenden Zylinder (34) befestigt ist, welcher so ausgebildet ist, daß er an dem zweiten Sockelelement (8) lösbar befestigbar ist, indem er durch die magnetische Kraft der stabförmigen Permanentmagnete (2) auf das zweite Sockelelement (8) gezogen wird.

2. Magnetvorrichtung mit zwei oder mit drei oder mit vier oder mit fünf oder mit sechs oder mit mehr als sechs stabförmigen Permanentmagneten (2), die alle zueinander jeweils parallel und magnetisch alle gleichsinnig orientiert angeordnet sind, wobei die Magnetvorrichtung die folgende Merkmale aufweist:
- ein erstes einteiliges Sockelelement (7) aus Stahl und ein zweites einteiliges Sockelelement (8) aus Stahl, die beide jeweils Vertiefungen (9) zur Aufnahme von Stirnseiten der stabförmigen Permanentmagnete (2) aufweisen, wobei in jedem der beiden Sockelelemente (7, 8) für jeden einzelnen der stabförmigen Permanentmagnete (2) jeweils eine separate Vertiefung (9) vorgesehen ist und die Permanentmagnete (2) in den beiden Sockelelementen (7, 8) derart gelagert sind, daß alle Permanentmagnete (2) mit ihrer jeweiligen Nordpolseite in dem ersten Sockelelement (7) und mit ihrer jeweiligen Südpolseite in dem zweiten Sockelelement (8) stecken, und
- ein erstes Stahlseil (3), mit einem ersten Ende und einem zweiten Ende, wobei das erste Ende des ersten Stahlseils (3) in einem ersten magnetisch leitenden Zylinder (33) befestigt ist, welcher so ausgebildet ist, daß er an dem ersten Sockelelement (7) lösbar befestigbar ist, indem er durch die magnetische Kraft der stabförmigen Permanentmagnete (2) auf das erste Sockelelement (7) gezogen wird, und das zweite Ende des ersten Stahlseils (3) in einem zweiten magnetisch leitenden Zylinder (34) befestigt ist, welcher so ausgebildet ist, daß er an dem zweiten Sockelelement (8) lösbar befestigbar ist, indem er durch die magnetische Kraft der stabförmigen Permanentmagnete (2) auf das zweite Sockelelement (8) gezogen wird.

3. Magnetvorrichtung nach Anspruch 1, **gekennzeichnet durch** ein Gehäuse (1) zur Aufnahme der stabförmigen Permanentmagnete (2) und der beiden Sockelelemente (7, 8), wobei das Gehäuse (1) auf einer seiner beiden Stirnseiten eine erste Öffnung zum Hineinstecken eines Endabschnittes des ersten Stahlseils (3) und auf einer anderen seiner beiden Stirnseiten eine zweite Öffnung zum Hineinstecken eines Endabschnittes des zweiten Stahlseils (4) aufweist, wobei die genannte erste Öffnung des Gehäuses (1) dem ersten Sockelelement (7) stirnseitig und bevorzugterweise axial zentriert gegenüberliegt und die genannte zweite Öffnung des Gehäuses (1) dem zweiten Sockelelement (8) stirnseitig und bevorzugterweise axial zentriert gegenüberliegt, und wobei ferner der erste magnetisch leitende Zylinder (33) so ausgebildet ist, daß er durch die erste Öffnung in dem Gehäuse (1) hindurch gesteckt werden kann, und der zweite magnetisch leitende Zylinder (34) so ausgebildet ist, daß er durch die zweite Öffnung in dem Gehäuse (1) hindurch gesteckt werden kann.

4. Magnetvorrichtung nach Anspruch 2, **gekennzeichnet durch** ein Gehäuse (1) zur Aufnahme der stabförmigen Permanentmagnete (2) und der beiden Sockelelemente (7, 8), wobei das Gehäuse (1) auf einer seiner beiden Stirnseiten eine erste Öffnung zum Hineinstecken eines Endabschnittes des ersten Stahlseils (3) und auf einer anderen seiner beiden Stirnseiten eine zweite Öffnung zum Hineinstecken eines anderen Endabschnittes des ersten Stahlseils (3) aufweist, wobei die genannte erste Öffnung des Gehäuses (1) dem ersten Sockelelement (7) stirnseitig und bevorzugterweise axial zentriert gegenüberliegt und die genannte zweite Öffnung des Gehäuses (1) dem zweiten Sockelelement (8) stirnseitig und bevorzugterweise axial zentriert gegenüberliegt, und wobei ferner der erste magnetisch leitende Zylinder (33) so ausgebildet ist, daß er durch die erste Öffnung in dem Gehäuse (1) hindurch gesteckt werden kann, und der zweite magnetisch leitende Zylinder (34) so ausgebildet ist, daß er durch die zweite Öffnung in dem Gehäuse (1) hindurch gesteckt werden kann.

5. Magnetvorrichtung mit zwei oder mit drei oder mit vier oder mit fünf oder mit sechs oder mit mehr als sechs stabförmigen Permanentmagneten (2), die alle zueinander jeweils parallel und magnetisch alle gleichsinnig orientiert angeordnet sind, wobei die Magnetvorrichtung die folgende Merkmale aufweist:
ein erstes einteiliges Sockelelement (7) aus Stahl und ein zweites einteiliges Sockelelement (8) aus Stahl, die beide jeweils Vertiefungen (9) zur Aufnahme von Stirnseiten der stabförmigen Permanentmagnete (2) aufweisen, wobei in jedem der beiden Sockelelemente (7, 8) für jeden einzelnen der stabförmigen Permanentmagnete (2) jeweils eine separate Vertiefung (9) vorgesehen ist und die Permanentmagnete (2) in den beiden Sockelelementen (7, 8) derart gelagert sind, daß alle Permanentmagnete (2) mit ihrer jeweiligen Nordpolseite in dem ersten Sockelelement (7) und mit ihrer jeweiligen Südpolseite in dem zweiten Sockelelement (8) stecken, wobei die Magnetvorrichtung ein erstes Stahlseil (3) und ein zweites Stahlseil (4) aufweist und
- das erste Sockelelement (7) eine von seiner Permanentmagnet-Aufnahmeseite abgewandten Seite ausgehende Bohrung oder Durchbohrung (10) zur Aufnahme eines Endabschnittes des ersten Stahlseils (3) aufweist und
- das zweite Sockelelement (8) eine von seiner Permanentmagnet-Aufnahmeseite abgewandten Seite ausgehende Bohrung oder Durchbohrung (11) zur Aufnahme eines Endabschnittes des zweiten Stahlseils (4) aufweist,
wobei der genannte Endabschnitt des ersten Stahlseils (3) in der Bohrung oder Durchbohrung (10) des ersten Sockelelements (7) steckt und der genannte Endabschnitt des zweiten Stahlseils (4) in der Bohrung oder Durchbohrung (11) des zweiten Sockelelements (8) steckt und jedes der beiden Sockelelemente (7, 8) ein Schraubenloch (12, 13) aufweist, durch das jeweils eine Schraube zum Fixieren des jeweiligen Stahlseils (3, 4) geführt ist, und wobei die beiden vorstehend genannten Bohrungen oder Durchbohrungen (10, 11) bevorzugterweise jeweils im Zentrum des jeweiligen Sockelelements (7, 8) angeordnet sind.

6. Magnetvorrichtung mit zwei oder mit drei oder mit vier oder mit fünf oder mit sechs oder mit mehr als sechs stabförmigen Permanentmagneten (2), die alle zueinander jeweils parallel und magnetisch alle gleichsinnig orientiert angeordnet sind, wobei die Magnetvorrichtung die folgende Merkmale aufweist:
ein erstes einteiliges Sockelelement (7) aus Stahl und ein zweites einteiliges Sockelelement (8) aus Stahl, die beide jeweils Vertiefungen (9) zur Aufnahme von Stirnseiten der stabförmigen Permanentmagnete (2) aufweisen, wobei in jedem der beiden Sockelelemente (7, 8) für jeden einzelnen der stabförmigen Permanentmagnete (2) jeweils eine separate Vertiefung (9) vorgesehen ist und die Permanentmagnete (2) in den beiden Sockelelementen (7, 8) derart gelagert sind, daß alle Permanentmagnete (2) mit ihrer jeweiligen Nordpolseite in dem ersten Sockelelement (7) und mit ihrer jeweiligen Südpolseite in dem zweiten Sockelelement (8) stecken, wobei die Magnetvorrichtung ein erstes Stahlseil (3) aufweist und
- das erste Sockelelement (7) eine von seiner Permanentmagnet-Aufnahmeseite abgewandten Seite ausgehende Bohrung oder Durchbohrung (10) zur Aufnahme eines Endabschnittes des ersten Stahlseils (3) aufweist und
- das zweite Sockelelement (8) eine von seiner Permanentmagnet-Aufnahmeseite abgewandten Seite ausgehende Bohrung oder Durchbohrung (11) zur Aufnahme eines anderen Endabschnittes des ersten Stahlseils (3) aufweist,
wobei der genannte eine Endabschnitt des ersten Stahlseils (3) in der Bohrung oder Durchbohrung (10) des ersten Sockelelements (7) steckt und der genannte andere Endabschnitt des ersten Stahlseils (3) in der Bohrung oder Durchbohrung (11) des zweiten Sockelelements (8) steckt und jedes der beiden Sockelelemente (7, 8) ein Schraubenloch (12, 13) aufweist, durch das jeweils eine Schraube zum Fixieren des ersten Stahlseils (3) geführt ist, und wobei die beiden vorstehend genannten Bohrungen oder Durchbohrungen (10, 11) bevorzugterweise jeweils im Zentrum des jeweiligen Sockelelements (7, 8) angeordnet sind.

7. Magnetvorrichtung nach Anspruch 5, **gekennzeichnet durch** ein Gehäuse (1) zur Aufnahme der stabförmigen Permanentmagnete (2) und der beiden Sockelelemente (7, 8), wobei sich das erste Stahlseil (3) durch eine erste Durchführung (5) in einer Wandung des Gehäuses (1) hindurch erstreckt und sich das zweite Stahlseil (4) durch eine zweite Durchführung (6) in der Wandung des Gehäuses (1) hindurch erstreckt.

8. Magnetvorrichtung nach Anspruch 6, **gekennzeichnet durch** ein Gehäuse (1) zur Aufnahme der stabförmigen Permanentmagnete (2) und der beiden Sockelelemente (7, 8), wobei sich das erste Stahlseil (3) sowohl durch eine erste Durchführung (5) in einer Wandung des Gehäuses (1) als auch durch eine zweite Durchführung (6) in der Wandung des Gehäuses (1) hindurch erstreckt.

9. Magnetvorrichtung nach einem der Ansprüche 1, 3, 5 oder 7, **dadurch gekennzeichnet, daß** das erste Stahlseil (3) an seinem zweiten Ende bzw. an seinem nicht in dem ersten Sockelelement (7) steckenden Ende mit einem ersten Griffelement (17) versehen ist und das zweite Stahlseil (4) an seinem zweiten Ende bzw. an seinem nicht in dem zweiten Sockelelement (8) steckenden Ende mit einem zweiten Griffelement (18) versehen ist, wobei in jedem einzelnen der beiden Griffelemente (17, 18) jeweils ein weiterer Permanentmagnet angeordnet ist.

10. Magnetvorrichtung nach Anspruch 9, **dadurch gekennzeichnet, daß** das erste Griffelement (17) an seiner freien Stirnseite konvex ausgebildet ist und das zweite Griffelement (18) an seiner freien Stirnseite konkav ausgebildet ist und die Form der beiden genannten freien Stirnseiten derart aufeinander abgestimmt ist, daß sie spaltfrei aneinander anliegen können.

11. Magnetvorrichtung nach einem der Ansprüche 1, 3, 5, 7, 9 oder 10, **dadurch gekennzeichnet, daß** die zweiten Enden der beiden Stahlseile (3, 4) bzw. die nicht in den Sockelelementen (7, 8) steckenden Enden der beiden Stahlseile (3, 4) oder, sofern vorhanden, die beiden Griffelemente (17, 18) aneinander anliegen, wobei die aneinander anliegenden Enden der beiden Stahlseile (3, 4) oder die beiden aneinander anliegenden Griffelemente (17, 18) bevorzugterweise mittels einer Sicherungseinrichtung (19) aneinander fixiert sind, wobei als Sicherungseinrichtung (19) bevorzugterweise ein Klebeband verwendet wird.

12. Magnetvorrichtung nach einem der vorhergehenden Ansprüche, **dadurch gekennzeichnet, daß** das erste Stahlseil (3) und, sofern vorhanden, auch das zweite Stahlseil (4) aus mehreren miteinander verdrillten Stahllitzen besteht.

13. Magnetvorrichtung nach einem der vorhergehenden Ansprüche, **dadurch gekennzeichnet, daß**
- jede Vertiefung (9) mindestens 4 mm oder mindestens 5 mm oder mindestens 6 mm oder mindestens 7 mm oder mindestens 8 mm oder mindestens 9 mm oder mindestens 10 mm oder mindestens 12 mm oder mindestens 14 mm oder mindestens 16 mm oder mindestens 18 mm oder mindestens 20 mm tief ist und
- jede Vertiefung (9) höchstens 50 mm oder höchstens 45 mm oder höchstens 40 mm oder höchstens 35 mm oder höchstens 30 mm tief ist.

14. Magnetvorrichtung nach einem der vorhergehenden Ansprüche, **dadurch gekennzeichnet, daß** jede Vertiefung (9) paßgenau auf den in ihr steckenden stabförmigen Permanentmagneten (2) abgestimmt ist, wobei das Spiel maximal 0,5 mm und bevorzugterweise maximal 0,2 mm beträgt.

15. Magnetvorrichtung nach einem der vorhergehenden Ansprüche, **gekennzeichnet durch** ein Gehäuse (1) zur Aufnahme der stabförmigen Permanentmagnete (2) und der beiden Sockelelemente (7, 8), wobei das Gehäuse (1) aus Nichteisenmetall oder aus Kunststoff oder aus Eisenmetall oder aus Stahl oder aus einer Nichteisenmetall-Legierung oder aus einer Metall-Legierung oder aus Aluminium besteht.

16. Magnetvorrichtung nach einem der vorhergehenden Ansprüche, **dadurch gekennzeichnet, daß**
- das erste Sockelelement (7) so ausgebildet ist, daß es auf der von seiner Permanentmagnet-Aufnahmeseite abgewandten Seite einen ersten Fortsatz (30) aufweist, dessen Durchmesser oder Breite kleiner ist als der Durchmesser oder die Breite des ersten Sockelelements (7) an seiner weitesten Stelle, wobei der erste Fortsatz (30) integraler Bestandteil des ersten Sockelelements (7) ist, so daß das erste Sockelelement (7) samt seinem ersten Fortsatz (30) ein einteiliges Stück ist, und/oder
- das zweite Sockelelement (8) so ausgebildet ist, daß es auf der von seiner Permanentmagnet-Aufnahmeseite abgewandten Seite einen zweiten Fortsatz (31) aufweist, dessen Durchmesser oder Breite kleiner ist als der Durchmesser oder die Breite des zweiten Sockelelements (8) an seiner weitesten Stelle, wobei der zweite Fortsatz (31) integraler Bestandteil des zweiten Sockelelements (8) ist, so daß das zweite Sockelelement (8) samt seinem zweiten Fortsatz (31) ein einteiliges Stück ist.

17. Magnetvorrichtung nach einem der vorhergehenden Ansprüche, **gekennzeichnet durch** einen ersten Lagerblock (14), auf dem das erste Sockelelement (7) fixiert ist, und/oder einen zweiten Lagerblock (15), auf dem das zweite Sockelelement (8) fixiert ist, wobei bevorzugterweise eine oder beide der vorstehend genannten Fixierungen mittels einer Schraube bzw. mittels jeweils einer Schraube erfolgt.

18. Magnetvorrichtung nach einem der vorhergehenden Ansprüche, **dadurch gekennzeichnet, daß** alle stabförmigen Permanentmagnete (2) radial im gleichen Abstand zum Zentrum des jeweiligen Sockelelements (7, 8) angeordnet sind.

19. Magnetvorrichtung nach einem der vorhergehenden Ansprüche, **dadurch gekennzeichnet, daß** sie genau drei stabförmige Permanentmagnete (2) aufweist, die radial im gleichen Abstand zum Zentrum des jeweiligen Sockelelements (7, 8) um jeweils 120° versetzt angeordnet sind.

20. Magnetvorrichtung nach einem der vorhergehenden Ansprüche, **dadurch gekennzeichnet, daß** alle oder mindestens einer der stabförmigen Permanentmagnete (2) zylinderförmig sind bzw. ist oder daß alle oder mindestens einer der stabförmigen Permanentmagnete (2) quaderförmig sind bzw. ist oder daß mindestens einer der stabförmigen Permanentmagnete (2) zylinderförmig ist und gleichzeitig mindestens ein anderer der stabförmigen Permanentmagnete (2) quaderförmig ist.

21. Magnetvorrichtung nach einem der vorhergehenden Ansprüche, **gekennzeichnet durch** eine Röhre (16), innerhalb der sich die stabförmigen Permanentmagnete (2) erstrecken und deren eine Stirnseite durch das erste Sockelelement (7) und deren andere Stirnseite durch das zweite Sockelelement (8) gedeckelt ist, wobei bevorzugterweise mindestens eines der beiden Sockelelemente (7, 8) so ausgebildet ist, daß es in die Röhre (16) hineinragt.

22. Magnetvorrichtung nach Anspruch 21, **dadurch gekennzeichnet, daß** die Röhre (16) aus einem Nichteisenmetall oder aus einer Nichteisenmetall-Legierung oder aus Kunststoff oder aus Nirosta oder aus Aluminium oder aus Messing besteht.

23. Magnetvorrichtung nach Anspruch 21 oder nach Anspruch 22, **dadurch gekennzeichnet, daß** die Röhre (16) an mindestens einem der beiden Sockelelemente (7, 8) mittels einer Schraube fixiert ist.

## Claims

1. A magnetic device with two or with three or with four or with five or with six or with more than six rod-shaped permanent magnets (2), which are all arranged parallel to one another and magnetically oriented all in the same direction, wherein the magnetic device has the following features:
- a first one-part holder element (7) made of steel and a second one-part holder element (8) made of steel, which both have indentations (9) for receiving end faces of the rod-shaped permanent magnets (2), a separate indentation (9) being provided in each of the two holder elements (7, 8) for each of the individual rod-shaped permanent magnets (2), and the permanent magnets (2) being mounted in the two holder elements (7, 8) in such a way that all permanent magnets (2) are inserted with their north pole end in the first holder element (7) and with their south pole end in the second holder element (8),
- a first steel cable (3), with a first end and a second end, the first end of the first steel cable (3) being fastened in a first magnetically conductive cylinder (33), which is configured such that it is releasably fastenable to the first holder element (7) by being pulled onto the first holder element (7) by the magnetic force of the rod-shaped permanent magnets (2), and
- a second steel cable (4) with a first end and a second end, the first end of the second steel cable (4) being fastened in a second magnetically conductive cylinder (34), which is configured such that it is releasably fastenable to the second holder element (8) by being pulled onto the second holder element (8) by the magnetic force of the rod-shaped permanent magnets (2).

2. A magnetic device with two or with three or with four or with five or with six or with more than six rod-shaped permanent magnets (2), which are all arranged parallel to one another and magnetically oriented all in the same direction, wherein the magnetic device has the following features:
- a first one-part holder element (7) made of steel and a second one-part holder element (8) made of steel, which both have indentations (9) for receiving end faces of the rod-shaped permanent magnets (2), a separate indentation (9) being provided in each of the two holder elements (7, 8) for each of the individual rod-shaped permanent magnets (2), and the permanent magnets (2) being mounted in the two holder elements (7, 8) in such a way that all permanent magnets (2) are inserted with their north pole end in the first holder element (7) and with their south pole end in the second holder element (8), and
- a first steel cable (3), with a first end and a second end, the first end of the first steel cable (3) being fastened in a first magnetically conductive cylinder (33), which is configured such that it is releasably fastenable to the first holder element (7) by being pulled onto the first holder element (7) by the magnetic force of the rod-shaped permanent magnets (2), and the second end of the first steel cable (3) being fastened in a second magnetically conductive cylinder (34), which is configured such that it is releasably fastenable to the second holder element (8) by being pulled onto the second holder element (8) by the magnetic force of the rod-shaped permanent magnets (2).

3. A magnetic device according to claim 1, **characterised by** a housing (1) for receiving the rod-shaped permanent magnets (2) and the two holder elements (7, 8), the housing (1) having, on one of its two end faces, a first opening for insertion of an end portion of the first steel cable (3) and, on another of its two end faces, a second opening for insertion of an end portion of the second steel cable (4), said first opening in the housing (1) being arranged opposite the end face of the first holder element (7), preferably in axially centred fashion, and said second opening in the housing (1) being arranged opposite the end face of the second holder element (8), preferably in axially centred fashion, furthermore the first magnetically conductive cylinder (33) being configured such that it can be inserted through the first opening in the housing (1), and the second magnetically conductive cylinder (34) being configured such that it can be inserted through the second opening in the housing (1).

4. A magnetic device according to claim 2, **characterised by** a housing (1) for receiving the rod-shaped permanent magnets (2) and the two holder elements (7, 8), the housing (1) having, on one of its two end faces, a first opening for insertion of an end portion of the first steel cable (3) and, on another of its two end faces, a second opening for insertion of another end portion of the first steel cable (3), said first opening in the housing (1) being arranged opposite the end face of the first holder element (7), preferably in axially centred fashion, and said second opening in the housing (1) being arranged opposite the end face of the second holder element (8), preferably in axially centred fashion, furthermore the first magnetically conductive cylinder (33) being configured such that it can be inserted through the first opening in the housing (1), and the second magnetically conductive cylinder (34) being configured such that it can be inserted through the second opening in the housing (1) .

5. A magnetic device with two or with three or with four or with five or with six or with more than six rod-shaped permanent magnets (2), which are all arranged parallel to one another and magnetically oriented all in the same direction, wherein the magnetic device has the following features:
- a first one-part holder element (7) made of steel and a second one-part holder element (8) made of steel, which both have indentations (9) for receiving end faces of the rod-shaped permanent magnets (2), a separate indentation (9) being provided in each of the two holder elements (7, 8) for each of the individual rod-shaped permanent magnets (2), and the permanent magnets (2) being mounted in the two holder elements (7, 8) in such a way that all permanent magnets (2) are inserted with their north pole end in the first holder element (7) and with their south pole end in the second holder element (8),
the magnetic device having a first steel cable (3) and a second steel cable (4) and
- the first holder element (7) having a bore or through-bore (10), starting from the side of the first holder element facing away from the permanent magnet-receiving side thereof, for receiving an end portion of the first steel cable (3) and
- the second holder element (8) having a bore or through-bore (11), starting from the side of the second holder element facing away from the permanent magnet-receiving side thereof, for receiving an end portion of the second steel cable (4),
said end portion of the first steel cable (3) being inserted into the bore or through-bore (10) in the first holder element (7) and said end portion of the second steel cable (4) being inserted into the bore or through-bore (11) in the second holder element (8), and each of the two holder elements (7, 8) having a screw hole (12, 13), through each of which a screw for fixing the corresponding steel cable (3, 4) is guided, the two aforesaid bores or through-bores (10, 11) preferably each being arranged in the centre of the corresponding holder element (7, 8).

6. A magnetic device with two or with three or with four or with five or with six or with more than six rod-shaped permanent magnets (2), which are all arranged parallel to one another and magnetically oriented all in the same direction, wherein the magnetic device has the following features:
- a first one-part holder element (7) made of steel and a second one-part holder element (8) made of steel, which both have indentations (9) for receiving end faces of the rod-shaped permanent magnets (2), a separate indentation (9) being provided in each of the two holder elements (7, 8) for each of the individual rod-shaped permanent magnets (2), and the permanent magnets (2) being mounted in the two holder elements (7, 8) in such a way that all permanent magnets (2) are inserted with their north pole end in the first holder element (7) and with their south pole end in the second holder element (8),
the magnetic device having a first steel cable (3) and
- the first holder element (7) having a bore or through-bore (10), starting from the side of the first holder element facing away from the permanent magnet-receiving side thereof, for receiving one end portion of the first steel cable (3) and
- the second holder element (8) having a bore or through-bore (11), starting from the side of the second holder element facing away from the permanent magnet-receiving side thereof, for receiving another end portion of the first steel cable (3),
said one end portion of the first steel cable (3) being inserted into the bore or through-bore (10) in the first holder element (7) and said other end portion of the first steel cable (3) being inserted into the bore or through-bore (11) in the second holder element (8), and each of the two holder elements (7, 8) having a screw hole (12, 13), through each of which a screw for fixing the first steel cable (3) is guided, the two aforesaid bores or through-bores (10, 11) preferably each being arranged in the centre of the corresponding holder element (7, 8).

7. A magnetic device according to claim 5, **characterised by** a housing (1) for receiving the rod-shaped permanent magnets (2) and the two holder elements (7, 8), the first steel cable (3) extending through a first feedthrough (5) in a wall of the housing (1), and the second steel cable (4) extending through a second feedthrough (6) in the wall of the housing (1).

8. A magnetic device according to claim 6, **characterised by** a housing (1) for receiving the rod-shaped permanent magnets (2) and the two holder elements (7, 8), the first steel cable (3) extending both through a first feedthrough (5) in a wall of the housing (1) and through a second feedthrough (6) in the wall of the housing (1).

9. A magnetic device according to one of claims 1, 3, 5 or 7, **characterised in that** the first steel cable (3) is provided at its second end or at its end not inserted in the first holder element (7) with a first grip element (17), and the second steel cable (4) is provided at its second end or at its end not inserted in the second holder element (8) with a second grip element (18), a further permanent magnet being arranged in each one of the two grip elements (17, 18).

10. A magnetic device according to claim 9, **characterised in that** the first grip element (17) is convex at its free end face and the second grip element (18) is concave at its free end face and the shapes of the two aforesaid free end faces are matched to one another in such a way that they can bear against one another without gaps.

11. A magnetic device according to one of claims 1, 3, 5, 7, 9 or 10, **characterised in that** the second ends of the two steel cables (3, 4) or the ends of the two steel cables (3, 4) not inserted into the holder elements (7, 8) or, if provided, the two grip elements (17, 18) bear against one another, the ends of the two steel cables (3, 4) bearing against one another or the two grip elements (17, 18) bearing against one another preferably being fixed to one another by way of a securing means (19), an adhesive tape preferably being used as securing means (19).

12. A magnetic device according to one of the preceding claims, **characterised in that** the first steel cable (3) and, if provided, also the second steel cable (4) consists of a plurality of steel wires twisted together.

13. A magnetic device according to one of the preceding claims, **characterised in that**
- each indentation (9) is at least 4 mm or at least 5 mm or at least 6 mm or at least 7 mm or at least 8 mm or at least 9 mm or at least 10 mm or at least 12 mm or at least 14 mm or at least 16 mm or at least 18 mm or at least 20 mm deep and
- each indentation (9) is at most 50 mm or at most 45 mm or at most 40 mm or at most 35 mm or at most 30 mm deep.

14. A magnetic device according to one of the preceding claims, **characterised in that** each indentation (9) is matched with an accurate fit to the rod-shaped permanent magnet (2) inserted thereinto, the play being at most 0.5 mm and preferably at most 0.2 mm.

15. A magnetic device according to one of the preceding claims, **characterised by** a housing (1) for receiving the rod-shaped permanent magnets (2) and the two holder elements (7, 8), the housing (1) consisting of a non-ferrous metal or of plastics or of ferrous metal or of steel or of a non-ferrous metal alloy or of a metal alloy or of aluminium.

16. A magnetic device according to one of the preceding claims, **characterised in that**
- the first holder element (7) is configured such that it has, on the side facing away from its permanent magnet-receiving side, a first extension (30), the diameter or width of which is smaller than the diameter or the width of the first holder element (7) at its widest point, the first extension (30) being an integral part of the first holder element (7), such that the first holder element (7) including its first extension (30) is a one-part piece, and/or
- the second holder element (8) is configured such that it has, on the side facing away from its permanent magnet-receiving side, a second extension (31), the diameter or width of which is smaller than the diameter or the width of the second holder element (8) at its widest point, the second extension (31) being an integral part of the second holder element (8), such that the second holder element (8) including its second extension (31) is a one-part piece.

17. A magnetic device according to one of the preceding claims, **characterised by** a first bearing block (14), on which the first holder element (7) is fixed, and/or a second bearing block (15), on which the second holder element (8) is fixed, one or both of said fixings preferably being achieved by means of a screw or, respectively, by means of a screw each.

18. A magnetic device according to one of the preceding claims, **characterised in that** all rod-shaped permanent magnets (2) are arranged radially at the same distance from the centre of their respective holder elements (7, 8).

19. A magnetic device according to one of the preceding claims, **characterised in that** it has exactly three rod-shaped permanent magnets (2), which are arranged radially at the same distance from the centre of their respective holder elements (7, 8), offset in each case by 120°.

20. A magnetic device according to one of the preceding claims, **characterised in that** all or at least one of the rod-shaped permanent magnets (2) are/is cylindrical, or **in that** all or at least one of the rod-shaped permanent magnets (2) are/is cuboidal, or **in that** at least one of the rod-shaped permanent magnets (2) is cylindrical and at the same time at least one other of the rod-shaped permanent magnets (2) is cuboidal.

21. A magnetic device according to one of the preceding claims, **characterised by** a tube (16), inside which the rod-shaped permanent magnets (2) extend with one end face thereof being covered by the first holder element (7) and the other end face thereof being covered by the second holder element (8), at least one of the two holder elements (7, 8) preferably being designed such that it protrudes into the tube (16).

22. A magnetic device according to claim 21, **characterised in that** the tube (16) is made of a non-ferrous metal or of a non-ferrous metal alloy or of plastic or of Nirosta or of aluminium or of brass.

23. A magnetic device according to claim 21 or according to claim 22, **characterised in that** the tube (16) is fixed to at least one of the two holder elements (7, 8) by means of a screw.

## Revendications

1. Dispositif à aimants avec deux ou avec trois ou avec quatre ou avec cinq ou avec six ou avec plus de six aimants permanents (2) en forme de barres, qui sont tous disposés parallèlement entre eux et tous orientés dans le même sens magnétique, dans lequel le dispositif à aimants présente les caractéristiques suivantes :
- un premier élément de socle d'une seule pièce (7) en acier et un deuxième élément de socle d'une seule pièce (8) en acier, qui présentent chacun tous les deux des cavités (9) pour le logement de faces frontales des aimants permanents (2) en forme de barres, dans lequel, dans chacun des deux éléments de socles (7, 8), une cavité (9) séparée est prévue pour chacun des aimants permanents (2) en forme de barres et les aimants permanents (2) sont logés dans les deux éléments de socles (7, 8) de façon à ce que tous les aimants permanents (2) s'emboîtent, avec leurs pôles nord respectifs dans le premier élément de socle (7) et, avec leurs pôles sud respectifs, dans le deuxième élément de socle (8),
- un premier câble en acier (3) avec une première extrémité et une deuxième extrémité, dans lequel la première extrémité du premier câble en acier (3) est fixée dans un premier cylindre magnétiquement conducteur (33), qui est conçu de façon à ce qu'il puisse être fixé de manière amovible au premier élément de socle (7), grâce au fait qu'il est attiré vers le premier élément de socle (7) par la force magnétique des aimants permanents (2) en forme de barres et
- un deuxième câble en acier (4) avec une première extrémité et une deuxième extrémité, dans lequel la première extrémité du deuxième câble en acier (4) est fixée dans un deuxième cylindre magnétiquement conducteur (34), qui est conçu de façon à ce qu'il puisse être fixé de manière amovible au deuxième élément de socle (8), grâce au fait qu'il est attiré vers le deuxième élément de socle (8) par la force magnétique des aimants permanents (2) en forme de barres.

2. Dispositif à aimants avec deux ou avec trois ou avec quatre ou avec cinq ou avec six ou avec plus de six aimants permanents (2) en forme de barres, qui sont tous disposés parallèlement entre eux et tous orientés dans le même sens magnétique, dans lequel le dispositif à aimants présente les caractéristiques suivantes :
- un premier élément de socle d'une seule pièce (7) en acier et un deuxième élément de socle d'une seule pièce (8) en acier, qui présentent chacun tous les deux des cavités (9) pour le logement de faces frontales des aimants permanents (2) en forme de barres, dans lequel, dans chacun des deux éléments de socles (7, 8), une cavité (9) séparée est prévue pour chacun des aimants permanents (2) en forme de barres et les aimants permanents (2) sont logés dans les deux éléments de socles (7, 8) de façon à ce que tous les aimants permanents (2) s'emboîtent, avec leurs pôles nord respectifs dans le premier élément de socle (7) et, avec leurs pôles sud respectifs, dans le deuxième élément de socle (8) et
- un premier câble en acier (3) avec une première extrémité et une deuxième extrémité, dans lequel la première extrémité du premier câble en acier (3) est fixée dans un premier cylindre magnétiquement conducteur (33), qui est conçu de façon à ce qu'il puisse être fixé de manière amovible au premier élément de socle (7), grâce au fait qu'il est attiré vers le premier élément de socle (7) par la force magnétique des aimants permanents (2) en forme de barres et la deuxième extrémité du premier câble en acier (3) est fixée dans un deuxième cylindre magnétiquement conducteur (34), qui est conçu de façon à ce qu'il puisse être fixé de manière amovible au deuxième élément de socle (8), grâce au fait qu'il est attiré vers le deuxième élément de socle (8) par la force magnétique des aimants permanents (2) en forme de barres.

3. Dispositif à aimants selon la revendication 1, **caractérisé par** un boîtier (1) pour le logement des aimants permanents (2) en forme de barres et des deux éléments de socles (7, 8), dans lequel le boîtier (1) comprend, sur une de ses deux faces frontales, une première ouverture pour l'insertion d'une portion d'extrémité du premier câble en acier (3) et, sur une autre de ses deux faces frontales, une deuxième ouverture pour l'insertion d'une portion d'extrémité du deuxième câble en acier (4), dans lequel la première ouverture mentionné du boîtier (1) fait face au premier élément de socle (7) côté frontal et de préférence de manière centrée axialement et la deuxième ouverture mentionnée du boîtier (1) fait face au deuxième élément de socle (8) côté frontal et de préférence de manière centrée axialement et dans lequel en outre le premier cylindre magnétiquement conducteur (33) est conçu de façon à ce qu'il puisse être emboîté à travers la première ouverture dans le boîtier (1) et le deuxième cylindre magnétiquement conducteur (34) est conçu de façon à ce qu'il puisse être emboîté à travers la deuxième ouverture dans le boîtier (1).

4. Dispositif à aimants selon la revendication 2, **caractérisé par** un boîtier (1) pour le logement des aimants permanents (2) en forme de barres et des deux éléments de socles (7, 8), dans lequel le boîtier (1) comprend, sur une de ses deux faces frontales, une première ouverture pour l'insertion d'une portion d'extrémité du premier câble en acier (3) et, sur une autre de ses deux faces frontales, une deuxième ouverture pour l'insertion d'une autre portion d'extrémité du premier câble en acier (3), dans lequel la première ouverture mentionné du boîtier (1) fait face au premier élément de socle (7) côté frontal et de préférence de manière centrée axialement et la deuxième ouverture mentionnée du boîtier (1) fait face au deuxième élément de socle (8) côté frontal et de préférence de manière centrée axialement et dans lequel en outre le premier cylindre magnétiquement conducteur (33) est conçu de façon à ce qu'il puisse être emboîté à travers la première ouverture dans le boîtier (1) et le deuxième cylindre magnétiquement conducteur (34) est conçu de façon à ce qu'il puisse être emboîté à travers la deuxième ouverture dans le boîtier (1).

5. Dispositif à aimants avec deux ou avec trois ou avec quatre ou avec cinq ou avec six ou avec plus de six aimants permanents (2) en forme de barres, qui sont tous disposés parallèlement entre eux et tous orientés dans le même sens magnétique, dans lequel le dispositif à aimants présente les caractéristiques suivantes :
un premier élément de socle d'une seule pièce (7) en acier et un deuxième élément de socle d'une seule pièce (8) en acier, qui présentent chacun tous les deux des cavités (9) pour le logement de faces frontales des aimants permanents (2) en forme de barres, dans lequel, dans chacun des deux éléments de socles (7, 8), une cavité (9) séparée est prévue pour chacun des aimants permanents (2) en forme de barres et les aimants permanents (2) sont logés dans les deux éléments de socles (7, 8) de façon à ce que tous les aimants permanents (2) s'emboîtent, avec leurs pôles nord respectifs dans le premier élément de socle (7) et, avec leurs pôles sud respectifs, dans le deuxième élément de socle (8),
dans lequel le dispositif à aimants comprend un premier câble en acier (3) et un deuxième câble en acier (4) et
- le premier élément de socle (7) comprend un perçage ou un perçage traversant (10) partant de son côté opposé au côté de logement des aimants permanents, pour le logement d'une portion d'extrémité du premier câble en acier (3) et
- le deuxième élément de socle (8) comprend un perçage ou un perçage traversant (11) partant de son côté opposé au côté de logement des aimants permanents, pour le logement d'une portion d'extrémité du deuxième câble en acier (4),
dans lequel la portion d'extrémité mentionnée du premier câble en acier (3) est emboîtée dans le perçage ou le perçage traversant (10) du premier élément de socle (7) et la portion d'extrémité mentionnée du deuxième câble en acier (4) est emboîtée dans le perçage ou le perçage traversant (11) du deuxième élément de socle (8) et chacun des deux éléments de socles (7, 8) comprend un trou taraudé (12, 13) à travers lequel est guidée une vis pour la fixation du câble en acier (3, 4) respectif et dans lequel les deux perçages ou perçages traversants (10, 11) précédemment mentionnés sont disposés de préférence chacun au centre de l'élément de socle (7, 8) respectif.

6. Dispositif à aimants avec deux ou avec trois ou avec quatre ou avec cinq ou avec six ou avec plus de six aimants permanents (2) en forme de barres, qui sont tous disposés parallèlement entre eux et tous orientés dans le même sens magnétique, dans lequel le dispositif à aimants présente les caractéristiques suivantes :
un premier élément de socle d'une seule pièce (7) en acier et un deuxième élément de socle d'une seule pièce (8) en acier, qui présentent chacun tous les deux des cavités (9) pour le logement de faces frontales des aimants permanents (2) en forme de barres, dans lequel, dans chacun des deux éléments de socles (7, 8), une cavité (9) séparée est prévue pour chacun des aimants permanents (2) en forme de barres et les aimants permanents (2) sont logés dans les deux éléments de socles (7, 8) de façon à ce que tous les aimants permanents (2) s'emboîtent, avec leurs pôles nord respectifs dans le premier élément de socle (7) et, avec leurs pôles sud respectifs, dans le deuxième élément de socle (8),
dans lequel le dispositif à aimants comprend un premier câble en acier (3) et
- le premier élément de socle (7) comprend un perçage ou un perçage traversant (10) partant de son côté opposé au côté de logement des aimants permanents, pour le logement d'une portion d'extrémité du premier câble en acier (3) et
- le deuxième élément de socle (8) comprend un perçage ou un perçage traversant (11) partant de son côté opposé au côté de logement des aimants permanents, pour le logement d'une autre portion d'extrémité du premier câble en acier (3),
dans lequel l' une portion d'extrémité mentionnée du premier câble en acier (3) est emboîtée dans le perçage ou le perçage traversant (10) du premier élément de socle (7) et l'autre portion d'extrémité mentionnée du premier câble en acier (3) est emboîtée dans le perçage ou le perçage traversant (11) du deuxième élément de socle (8) et chacun des deux éléments de socles (7, 8) comprend un trou fileté (12, 13) à travers lequel est guidée une vis pour la fixation du premier câble en acier (3) et dans lequel les deux perçages ou perçages traversants (10, 11) précédemment mentionnés sont disposés de préférence au centre de l'élément de socle (7, 8) respectif.

7. Dispositif à aimants selon la revendication 5, **caractérisé par** un boîtier (1) pour le logement des aimants permanents (2) en forme de barres et des deux éléments de socles (7, 8), dans lequel le premier câble en acier (3) s'étend à travers un premier passage (5) dans une paroi du boîtier (1) et le deuxième câble en acier (4) s'étend à travers un deuxième passage (6) dans la paroi du boîtier (1).

8. Dispositif à aimants selon la revendication 6, **caractérisé par** un boîtier (1) pour le logement des aimants permanents (2) en forme de barres et des deux éléments de socles (7, 8), dans lequel le premier câble en acier (3) s'étend aussi bien à travers un premier passage (5) dans une paroi du boîtier (1) qu'à travers un deuxième passage (6) dans la paroi du boîtier (1).

9. Dispositif à aimants selon l'une des revendications 1, 3, 5 ou 7, **caractérisé en ce que** le premier câble en acier (3) est muni, au niveau de sa deuxième extrémité resp. au niveau de son extrémité non emboîtée dans le premier élément de socle (7), avec un premier élément de préhension (17) et le deuxième câble en acier (4) est muni, au niveau de sa deuxième extrémité resp. au niveau de son extrémité non emboîtée dans le deuxième élément de socle (8), avec un deuxième élément de préhension (18), dans lequel, dans chacun des deux éléments de préhension (17, 18) est disposé un autre aimant permanent.

10. Dispositif à aimants selon la revendication 9, **caractérisé en ce que** le premier élément de préhension (17) est réalisé de manière convexe au niveau de sa face frontale libre et le deuxième élément de préhension (18) est réalisé de manière concave au niveau de sa face frontale libre et la forme des deux faces frontales libres mentionnées est réalisée de façon qu'elles puissent s'appuyer l'une contre l'autre sans interstice.

11. Dispositif à aimants selon l'une des revendications 1, 3, 5, 7, 9 ou 10, **caractérisé en ce que** les deuxièmes extrémités des deux câbles en acier (3, 4) resp. les extrémités des deux câbles en acier (3, 4) non emboîtées dans les éléments de socles (7, 8) ou, s'ils existent, les deux éléments de préhension (17, 18) s'appuient l'un contre l'autre, dans lequel les extrémités appuyées l'une contre l'autre des deux câbles en acier (3, 4) ou les deux éléments de préhension (17, 18) appuyés l'un contre l'autre sont fixés entre eux de préférence au moyen d'un dispositif de fixation (19), dans lequel le dispositif de fixation (19) utilisé peut être de préférence une bande adhésive.

12. Dispositif à aimants selon l'une des revendications précédentes, **caractérisé en ce que** le premier câble en acier (3) et, s'il existe, également le deuxième câble en acier (4) est constitué de plusieurs torons en acier torsadés entre eux.

13. Dispositif à aimants selon l'une des revendications précédentes, **caractérisé en ce que**
- chaque cavité (9) présente une profondeur d'au moins 4 mm ou d'au moins 5 mm ou d'au moins 6 mm ou d'au moins 7 mm ou d'au moins 8 mm ou d'au moins 9 mm ou d'au moins 10 mm ou d'au moins 12 mm ou d'au moins 14 mm ou d'au moins 16 mm ou d'au moins 18 mm ou d'au moins 20 mm et
- chaque cavité (9) présente une profondeur de 50 mm maximum ou de 45 mm maximum ou de 40 mm maximum ou de 35 mm maximum ou de 30 mm maximum.

14. Dispositif à aimants selon l'une des revendications précédentes, **caractérisé en ce que** chaque cavité (9) est adaptée de manière précise aux aimants permanents (2) en forme de barres emboîtés dans celle-ci, dans lequel le jeu est de 0,5 mm maximum et de préférence de 0,2 mm maximum.

15. Dispositif à aimants selon l'une des revendications précédentes, **caractérisé par** un boîtier (1) pour le logement des aimants permanents (2) en forme de barres et des deux éléments de socles (7, 8), dans lequel le boîtier (1) est constitué d'un métal non ferreux ou d'une matière plastique ou d'un métal ferreux ou d'acier ou d'un alliage métallique non ferreux ou d'un alliage métallique ou d'aluminium.

16. Dispositif à aimants selon l'une des revendications précédentes, **caractérisé en ce que**
- le premier élément de socle (7) est conçu de façon à ce qu'il comprenne, sur son côté opposé au côté de logement des aimants permanents, un premier prolongement (30), dont le diamètre ou la largeur est inférieure au diamètre ou à la largeur du premier élément de socle (7) à son endroit le plus large, dans lequel le premier prolongement (30) fait partie intégrante du premier élément de socle (7), de façon ce que le premier élément de socle (7) forme une seule pièce avec son premier prolongement (30) et/ou
- le deuxième élément de socle (8) est conçu de façon à ce qu'il comprenne, sur son côté opposé au côté de logement des aimants permanents, un deuxième prolongement (31), dont le diamètre ou la largeur est inférieure au diamètre ou à la largeur du deuxième élément de socle (8) à son endroit le plus large, dans lequel le deuxième prolongement (31) fait partie intégrante du deuxième élément de socle (8), de façon à ce que le deuxième élément de socle (8) forme une seule pièce avec son deuxième prolongement (31).

17. Dispositif à aimants selon l'une des revendications précédentes, **caractérisé par** un premier bloc de palier (14) sur lequel le premier élément de socle (7) est fixé et/ou un deuxième bloc de palier (15), sur lequel le deuxième élément de socle (8) est fixé, dans lequel, de préférence, une ou les deux fixations précédemment mentionnées sont effectuées au moyen d'une vis resp. chacune au moyen d'une vis.

18. Dispositif à aimants selon l'une des revendications précédentes, **caractérisé en ce que** tous les aimants permanents (2) en forme de barres sont disposés radialement à la même distance du centre de l'élément de socle (7, 8) respectif.

19. Dispositif à aimants selon l'une des revendications précédentes, **caractérisé en ce qu'**il comprend exactement trois aimants permanents (2) en forme de barres, qui sont disposés radialement à la même distance du centre de l'élément de socle (7, 8) respectif, de manière décalée de 120° entre eux.

20. Dispositif à aimants selon l'une des revendications précédentes, **caractérisé en ce que** tous ou au moins un des aimants permanents (2) en forme de barres présentent resp. présente une forme cylindrique ou **en ce que** tous ou au moins un des aimants permanents (2) en forme de barres présentent resp. présente une forme parallélépipédique ou **en ce qu'**au moins un des aimants permanents (2) en forme de barres présente une forme cylindrique et en même temps au moins un autre des aimants permanents (2) en forme de barres présente une forme parallélépipédique.

21. Dispositif à aimants selon l'une des revendications précédentes, **caractérisé par** un tube (16) à l'intérieur duquel s'étendent les aimants permanents (2) en forme de barres et dont une face frontale est recouverte par le premier élément de socle (7) et dont l'autre face frontale est recouverte par le deuxième élément de socle (8), dans lequel, de préférence, au moins un des deux éléments de socle (7, 8) est conçu de façon à dépasser dans le tube (16).

22. Dispositif à aimants selon la revendication 21, **caractérisé en ce que** le tube (16) est constitué d'un métal non ferreux ou d'un alliage de métal non ferreux ou d'une matière plastique ou de Nirosta ou d'aluminium ou de laiton.

23. Dispositif à aimants selon la revendication 21 ou selon la revendication 22, **caractérisé en ce que** le tube (16) est fixé à au moins un des deux éléments de socle (7, 8) au moyen d'une vis.
